# EUROPEAN PATENT APPLICATION

(11) **EP 2 913 674 A1**
(43) Date of publication of application: **02.09.2015**
(21) Application number: 14157389.9
(22) Date of filing: 28.02.2014
(51) Int. Cl.: G01N 33/68

(54) **Stepwise assembled capture compounds comprising a cleavable function and method for isolating and/or characterizing biomolecules or biomolecule fragments, in particular proteins or protein fragments, of complex mixtures**

(71) Applicant: Caprotec Bioanalytics GmbH, 12489 Berlin (DE)
(72) Inventor: Köster, Hubert, 6922 Morcote (CH); Dreger, Mathias, 10405 Berlin (DE); Michaelis, Simon, 10997 Berlin (DE); Lenz, Thomas, 12524 Berlin (DE); Milic, Jelena, 10997 Berlin (DE); Sefkow, Michael, 10551 Berlin (DE)
(74) Representative: Bolsinger, Jens Roland

(57) **Abstract**

The invention relates to a process for isolating and/or characterising biomolecules and/or biomolecule fragments, in particular proteins and/or protein fragments, comprising the steps:
provision of an immobilized compound IC through the interaction of a capture compound CC and the target compound T and a carrier compound CCB, carrying out a division step, in which a cleavable function F is cleaved, and a division compound DC is produced, and
isolation and/or characterisation of said division compound DC. The invention relates further to a carrier compound CCB, a capture compound CC, a precursor compound PC and a division compound DC.

## Description

### FIELD OF THE INVENTION

The present invention relates to compounds, reagent combinations and methods useful in isolating and/or characterizing biomolecules or biomolecule fragments, in particular proteins or protein fragments of complex mixtures such as cell lysates or living cells.

### BACKGROUND OF THE INVENTION

Proteins form the important structural and functional machinery of the cell, and are the molecular entities with which nearly all of today's marketed drugs interact. Proteins are thus drug targets. Furthermore, identifying "non-target" proteins with which the drug interacts to trigger side effects has been especially elusive. It is believed that side effects of many drugs could be diminished with a greater understanding of the mechanism of action involving their target and the non-target proteins, in particular by identifying the drug binding site and/or the cross link site, more particularly the drug binding site, of such proteins.

Thus, there is a need to reduce time and costs of drug development by
(a) accelerating the hit-to-drug selection process by filtering out those hits likely to trigger side effects and
(b) re-engineering drug chemical structure based on the knowledge of drug-target and drug-non-target interactions, reducing or eliminating the undesired interactions.

There is also a need to develop technologies for analysis of the proteome that allow scaling up to industrial levels with the features of an industrial process: high accuracy, reproducibility and flexibility in that the process is high-throughput, automatable and cost-effective. There is a need to develop technologies that permit probing and identification of proteins in their native conformation using automated protocols and systems therefore.

However, these needs are not only limited to drug development. Many other bioactive small compounds (e.g. natural products or herbicides etc.) interact with proteins. A deeper understanding of such an interaction is essential.

Tri-functional compounds for selective reaction with proteins are known from the WO 2004/064972 A2. These so called capture compounds described therein comprise three essential functionalities X, Y and Q linked through a central core Z. These functions impart the selective affinity targeting of the capture compound for certain target proteins (selectivity function or moiety Y), the light-mediated covalent linkage of the capture compound to the target protein (reactivity function or moiety X), and the subsequent removal or isolation of the capture-compound-protein complex from the mixture by a sorting function Q.

Different methods for analyzing or identifying the isolated ("captured") proteins can be employed. For high-throughput analysis, mass spectrometry has proven effective. The combination of capture compound isolation and mass spectrometric analysis is referred to as CCMS (Capture Compound Mass Spectrometry). The Capture Compound Mass Spectrometry (CCMS) is a novel platform technology combining two basic technologies, named photo-crosslinking of proteins with small, reactive molecules and liquid chromatography - mass spectrometry (LC-MS).

The isolation step in CCMS involves the equilibration of the capture compound with a complex mixture of proteins, for example a cell homogenate, a cell lysate, an isolated organ, a specific subcellular fraction/organelle, such as nuclei and mitochondria or living cells with intact cellular architecture, or an organelle. This first contacting step is performed in the absence of light able to activate the reactivity function X. The capture compound selectively associates (non-covalently) with certain proteins to which the selectivity function has a high affinity. One example is a capture compound having a pharmaceutical drug as Y, which will then specifically interact with drug target proteins and, in addition, any protein that the drug does also interact with specifically with high affinity but is not designed to do so ("off-targets"). Once equilibration has occurred, the sample is irradiated with light of a wavelength sufficient to activate the reactivity function X, which immediately reacts with any suitable neighboring group, effectively linking the target or off-target protein to the capture compound. The exact position in the amino acid sequences of target and off-target proteins, to which the capture compound is covalently linked, is, if precisely identified, informative on the small molecule (drug) binding site of the target or off-target protein itself.

Subsequently, the capture compound, with any attached protein, is removed from the mixture by contacting the mixture with a ligand for the sorting function that allows for removal of Q-containing molecules by attachment to a surface. One prominent example of Q is biotin, which can mediate removal of biotinylated molecules via highly stable association to streptavidin bound to magnetic particles or surfaces ("matrices").

During CCMS, matrices to which captured proteins are attached, bind proteins non-specifically to a certain extent. These non-specifically bound proteins form a background "noise" appearing also in control experiments, potentially drowning out signals from proteins that have bound specifically in small quantity.

The WO 2012/156377 A1 describes a possibility to reduce a potential drowning of signals. Aside from the three essential functionalities X (reactivity function), Y (selectivity function) and Q (sorting function), which are linked by linker moieties S to a central core Z, the capture compound comprises further a cleavable function F (which is cleavable by light of a certain wavelength) in the proximity of Q. Thus, the captured proteins can be removed and separated from the matrices (sorting function Q) prior to an analysis. Thus, the background "noise" from said non-specifically bound proteins can be reduced.

However, the remaining capture compound, to which the proteins are attached, comprises still a significant size, due to the remaining reactivity function X, selectivity function Y and the remaining necessary linkers S connected to the before mentioned functions. If the analysis is achieved by mass spectrometry, "bigger" molecules will generally complicate an analysis, since said "big" molecules are more prone to collapse into a multiplicity of fragments than "smaller" molecules, which will hinder the analysis and identification. In particular, the determination of the respective crosslink site is complicated or even - depending on the molecule and captured protein - impossible. In addition, the remaining part of the capture compound after cleavage and cross linked to the affinity-captured protein should be stable under mass spectrometric detection or fragmenting in a reproducible way to only a few defined fragment ions permitting identifying the crosslink peptide after protein digestion and mass spectrometry.

The objective of the present invention is to provide methods, compounds and reagents to overcome the stated problems of the state of the art. This objective is attained by the subject-matter of the independent claims.

### TERMS AND DEFINITIONS

As used herein the term "characterising," refers to the identification of captured target proteins or protein fragments (or biomolecules or biomolecules fragments) as well as to the determination or identification of certain functionalities or aspects of the captured proteins (or protein fragments), in particular the determination or identification of the respective cross-link site of said target or off-target proteins (protein fragments).

As used herein the term "reactivity function" refers to a moiety that can be stimulated (activated) to generate a reactive species such as a nitrene, a carbene or a radical by irradiation such as visible light or UV light. Such a reactive species is capable of quickly forming a covalent bond with a range of suitable partners, for example by addition to a C=C-double bond, or by insertion into a O-H, S-H, N-H or C-H bond. In other words, the reactivity function includes groups that specifically react or interact after activation with functionalities on the surface of a protein such as hydroxyl, amine, amide, sulfide and carboxylic acid groups or interacts with the active site of enzymes yielding covalent bond between the target or off-target protein and the reactivity function. Those skilled in the art can select from a library of functionalities to accomplish this reaction.

As used herein the term "activated function" refers to a structural element of the reactivity function derived from activation of the reactivity function by irradiation and the subsequent forming of a covalent bond to the target compound (proteins or protein fragments).

As used herein the term "selectivity function" refers to a variety of groups, which are capable of interacting non-covalently (selective interaction) with target compounds (proteins) based on affinity. The selectivity function in principle may be any small molecular moiety with a highly selective affinity to a target (or off-target) protein (or protein fragment) in the micromolar, nanomolar or sub-nanomolar range resulting in a functional enrichment based on the selective affinity of the selectivity function to the respective proteins in the protein mixture.

As used herein the term "drug binding site" is the site of a target compound (in particular a protein), which comprises the selective interaction of the selectivity function Y with target compound (in particular the protein).

As used herein the term "cross link site" refers to the site of a target compound (in particular a protein), which comprises the covalent bonding of the reactivity function X with the respective target compound (in particular the protein). Depending on the used capture compound - due to the flexible nature of the capture compound - there may be several possible cross linking sites on the target compound, which are situated - due to the structure of the capture compound - in proximity to the drug binding site.

As used herein the term "cleavable function" refers to a cleavable bond or moiety that is cleaved or cleavable under the specific conditions, such as chemically, enzymatically or by irradiation. A cleavable function is a moiety that can be selectively cleaved without affecting or altering the composition of the other portions of the respective compound of interest. For example, a cleavable moiety of the compounds provided herein is one that can be cleaved by chemical, enzymatic, photolytic, or other means without affecting or altering the composition (e. g. the chemical composition) of a captured protein. Non limiting examples are diarylazo, dithio or silyloxy groups. These moieties can be chemically cleaved in the presence of biopolymers using either reducing agents (e.g. sodium dithionite for azo groups, organothiols for disulfide bridges) or fluorides (for silyloxy groups). A function cleavable by irradiation may also be used. However, in this case, the cleavable function has to be chosen in such a way, that the necessary wavelength for the cleaving step is significantly different from the necessary wavelength for the activation of the reactivity function. As used herein the term "fragment part" of the cleavable function refers to the remaining structural element on the target site after a cleaving step was applied.

As used herein the term "bioactive" refers to a compound which is capable of interaction with or effect on any living matter, in particular cells and tissue in the human body or any other living being. Under this definition falls also a pharmacological activity, which refers to compounds with beneficial or adverse effects on any living matter, in particular to cells and tissue in the human body, animals, insects, plants, etc.. Bioactive compounds include - by way of non-limiting example - pharmaceutical drugs to treat humans and animals, insecticides, herbicides, pesticides and fungicides.

As used herein the term "small molecule" refers to a moiety of a molecular mass of less than 1500 Daltons, in particular a moiety of a molecular mass of less than 1000 Daltons, more particularly a moiety of a molecular mass of less than 500 Daltons.

As used herein the term "pharmaceutical drug" refers to a compound, which has properties for use in the medical diagnosis, cure, treatment, or prevention of disease, in particular any compound which may be used in view of restoring, correcting or modifying physiological functions by exerting a pharmacological, immunological or metabolic action, or to making a medical diagnosis

As used herein the term "drug metabolite" refers to any compound that is formed after transformation of a pharmaceutical drug following its metabolism in the body that results in a different molecule that may be responsible for adverse side (off-target) effects.

As used herein the term "prodrug" refers to a compound, which is formulated deliberately so it will break down inside the body to form the active drug.

As used herein the term "drug fragment" refers to a portion or moiety of a pharmaceutical drug, a drug development candidate or a drug metabolite or a prodrug.

As used herein the term "natural product" refers to a compound produced by a living organism and which comprises a pharmacological or biological activity that can be of therapeutic benefit in treating diseases.

As used herein the term "macroscopic carrier" refers to an element, which can be separated due to its physical properties (e.g. by centrifugation, filtering, use of a magnetic field etc.) from a mixture of components and comprises a non-gaseous, non- liquid material having a surface. Thus, a macroscopic carrier can be - by way of non-limiting example - a flat surface constructed, for example, of glass, silicon, metal, plastic or a composite; or can be in the form of a bead such as a silica gel, a controlled pore glass, a magnetic or cellulose bead; or a pin, including an array of pins suitable for combinatorial synthesis or analysis, a hollow fibre, a microtiter plate.

As used herein the term "spacer moieties" or "spacer moiety" refers to a covalently connected straight chain or a ring structure containing carbon, phosphorous, sulphur, silicon, nitrogen and/or oxygen atoms connecting one moiety to another moiety providing a certain distance between these moieties. The spacer moieties may comprise further substituent groups, which are not involved in providing said distance.

As used herein the term "linking function" refers to a functional group capable of forming selectively a covalent bond with another functional group under reaction conditions, which are not leading to a covalent reaction of said linking functions with natural occurring polypeptides, in particular with proteins. Thus, the two linking function allow to covalently connect one moiety comprising on linking function with another moiety comprising the other linking function without a reaction with the respective target compounds (proteins). Non-limiting examples are compounds, which are used in the so called "click chemistry".

As used herein the term "biological sample" refers to compositions containing biomolecules (in particular proteins) to be isolated and identified. For the purposes herein, sample refers to anything which can contain a target compound (in particular a protein). Thus, biological samples include (e.g. any material obtained from a source originating from a living being (e. g. human, animal, plant, bacteria, fungi or protist). The biological sample can be in any form, including solid materials (e. g. tissue, cell pellets and biopsies, tissues from cadavers) and biological fluids (e.g. urine, blood, saliva, amniotic fluid and mouth wash (containing buccal cells).

As used herein the term "biomolecules" refers to any molecule that is produced by a living organism, including large macromolecules such as proteins, polysaccharides, lipids, and nucleic acids.

As used herein the term "digestive step" refers to a procedure, in which a protein is cut enzymatically into a limited number of defined shorter fragments (peptides). These fragments allow for the identification of the protein with their characteristic mass and pattern. The serine protease trypsin can be used - by way of a non limiting example - as an enzyme.

As used herein the term "substituted" refers to the addition of a substituent group to a parent compound.

As used herein the term "substituent group" can be protected or unprotected and can be added to one available site or to many available sites in a parent compound. Substituent groups may also be further substituted with other substituent groups and may be attached directly or by a linking group such as an alkyl, an amide or hydrocarbyl group to a parent compound."Substituent groups" amenable herein include, without limitation, halogen, oxygen, nitrogen, sulphur, hydroxyl, alkyl, alkenyl, alkynyl, acyl (-C(O)R^{a}), carboxyl (-C(O)OR^{a}), aliphatic groups, alicyclic groups, alkoxy, substituted oxy (-OR^{a}), aryl, aralkyl, heterocyclic radical, heteroaryl, heteroarylalkyl, amino (-N(R^{b})(R^{c})), imino(=NR^{b}), amido (-C(O)N(R^{b})(R^{c}) or-N(R^{b})C(O)R^{a}), hydrazine derivates (-C(NH)NR^{a}R^{b}), triazole, tetrazole (CN₄H₂), azido (-N₃), nitro (-NO₂), cyano (-CN), isocyano (-NC), cyanato (-OCN), isocyanato (-NCO), thiocyanato (-SCN); isothio-cyanato (-NCS); carbamido (-OC(O)N(R^{b})(R^{c}) or -(R^{b})C(O)OR^{a}), thiol (-SR^{b}), sulfinyl (-S(O)R^{b}), sulfonyl (-S(O)₂R^{b}), sulfonamidyl (-S(O)₂N(R^{b})(R^{c})or -N(R^{b})S(O)₂R^{b}) and fluorinated compounds -CF₃, -OCF₃, -SCF₃, -SOCF₃ or -SO₂CF₃. Wherein each R^{a}, R^{b} and R^{c} is, independently, H or a further substituent group with a preferred list including without limitation, H, alkyl, alkenyl, alkynyl, aliphatic, alkoxy, acyl, aryl, heteroaryl, alicyclic, heterocyclic and heteroarylalkyl.

As used herein the term "alkyl," refers to a saturated straight or branched hydrocarbon moiety containing up to 20, particularly up to 10 carbon atoms. Examples of alkyl groups include, without limitation, methyl, ethyl, propyl, butyl, isopropyl, n-hexyl, octyl, decyl and the like. Alkyl groups typically include from 1 to about 20 carbon atoms, particularly from 1 to about 10 carbon atoms.

As used herein the term "aliphatic chain" refers to a straight or branched chain of carbon atoms derived from alkanes, alkenes, and alkynes. As used herein the term "heteroalkylchain" refers to an aliphatic chain in which at least one carbon atom is replaced with an oxygen, a nitrogen or a sulphur atom.

As used herein the term "cycloalkyl" refers to an interconnected alkyl group forming a ring structure containing 3 to 8, particularly 5 to 6 carbon atoms. Examples of cycloalkyl groups include, without limitation, cyclopropane, cyclopentane, cyclohexane or cyclooctane and the like. Cycloalkyl groups typically include from 5 to 6 carbon atoms (C₃-C₆ cycloalkyl). The term "unsaturated cycloalkyl" refers to a cycloalkyl group comprising at least one double or triple bond. Cycloalkyl groups as used herein may optionally include further substituent groups.

As used herein the term "unsaturated heterocycle" refers to cycloalkyl compounds in which at least one carbon atom is replaced with an oxygen, a nitrogen or a sulphur atom forming a ring structure. Said ring structure comprising at least one double or triple bond. Unsaturated heterocycle groups as used herein may optionally include further substituent groups.

As used herein the term "alkenyl," refers to a straight or branched hydrocarbon chain moiety containing up to 20 carbon atoms and having at least one carbon-carbon double bond. Examples of alkenyl groups include, without limitation, ethenyl, propenyl, butenyl, 1-methyl-2-buten-1-yl, dienes such as 1,3-butadiene and the like. Alkenyl groups typically include from 2 to about 20 carbon atoms, more typically from 2 to about 10 carbon atoms. Alkenyl groups as used herein may optionally include further substituent groups.

As used herein the term "alkynyl," refers to a straight or branched hydrocarbon moiety containing up to 20 carbon atoms and having at least one carbon-carbon triple bond. Examples of alkynyl groups include, without limitation, ethynyl, 1-propynyl, 1-butynyl, and the like. Alkynyl groups typically include from 2 to about 20 carbon atoms, more typically from 2 to about 10 carbon atoms. Alkynyl groups as used herein may optionally include further substituent groups.

As used herein the term "aryl" refers to a hydrocarbon with alternating double and single bonds between the carbon atoms forming a ring structure (in the following also "aromatic hydrocarbon"). The term "heteroaryl" refers to aryl compounds in which at least one carbon atom is replaced with an oxygen, a nitrogen or a sulphur atom. Aryl or hetero aryl groups as used herein may optionally include further substituent groups.

As used herein the term "protein capture compound PCC" refers - due to simplicity reasons - not only to a capture compound CC comprising an interaction with a protein P, as discussed below, but also to a capture compound CC comprising an interaction with a biomolecule BM, as depicted for example in formula 5. If not stated otherwise a "protein capture compound PCC" comprises the structural elements BM or P, however, P is preferred. The same applies to the "protein fragment capture compound PFCC".

### SUMMARY OF THE INVENTION

According to a first aspect of the invention provided herein is a process for isolating and/or characterising biomolecules and/or biomolecule fragments, in particular proteins and/or protein fragments, comprising the steps:
a. provision of an immobilized compound IC of the general formula 1 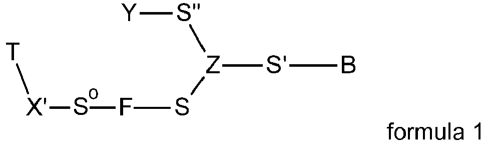 wherein
   - T is a target compound comprising, in particular essentially consisting of, a biomolecule BM or a biomolecule fragment BMF, more particularly a protein P or a protein fragment PF,
   - X' is the chemical structure resulting from activation of the reactivity function X by irradiation and the subsequent forming of a covalent bond to the target compound T,
   - F is a cleavable function,
   - Y is a selectivity function selected from the group comprising a bioactive small molecule,
   - Z is carbon, nitrogen, phosphorus, silicon, sulfonium, cycloalkyl, unsaturated cycloalkyl, unsaturated heterocycloalkyl or heteroaryl,
   - B is a macroscopic carrier, which is capable of being separated from a medium by means of his physical properties,
   - S°, S, S' and S" are spacer moieties, each of which represents, independently of the others, a bridge between X' and F, Z and F, Z and B or Z and Y, facilitated by covalent bonds of carbon, nitrogen, oxygen, sulphur, silicon and/or phosphorous atoms, wherein S°, S and S" are optional, and wherein S' comprises the spacer moieties S'" and S^{iv} of formula 2 and 3 and additionally the structural element derived from the reaction of the linking functions D and E;
      through the interaction of
      i. a capture compound CC of the general formula 2 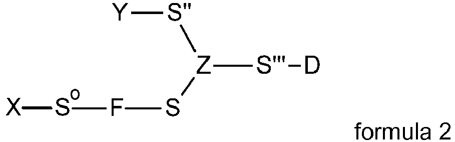 wherein
         - X is a reactivity function which can be activated by irradiation and can form after said activation a covalent bond to the target compound T, and
         - F, Y, Z, S, S° and S" each have the meanings defined previously, and
         - S'" is a spacer moiety representing a bridge between Z and D, facilitated by covalent bonds of carbon, nitrogen, oxygen, sulphur, silicon and/or phosphorous atoms, wherein S'" is optional, and
         - D is a first linking function capable of forming a covalent bond selectively with a second linking function E under reaction conditions not leading to a covalent reaction of D or E with natural occurring polypeptides, in particular with proteins;
      ii. the target compound T; and
      iii. a carrier compound CCB of the general formula 3 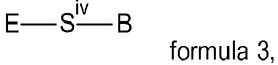 wherein
         - S^{iV} is a spacer moiety representing a bridge between E and B facilitated by covalent bonds of carbon, nitrogen, oxygen, sulphur, silicon and/or phosphorous atoms, wherein S^{iv} is optional,
         - E is a second linking function capable of forming a covalent bond selectively with a first linking function D under reaction conditions not leading to a covalent reaction of E or D with natural occurring polypeptides, in particular with protein
         - B has the meaning defined previously, and
b. carrying out a division step, in which the cleavable function F is cleaved, and a division compound DC of the general formula 4 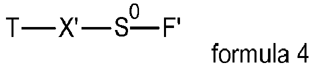 is produced, wherein
   - T, S⁰ and X' have the meanings defined previously, and
   - F' is the chemical structure resulting from a cleavage reaction of the cleavable function F; and
c. isolation and/or characterisation of said division compound DC.

Any process of the invention is an ex-situ process.

According to a second aspect of the invention provided herein is a compound, particularly a compound for carrying out a process according to the first aspect of the invention, of the general formula 3, 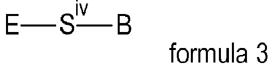 wherein
- S^{iV} comprises a polyethylene glycol group,
- B is a macroscopic carrier, which is capable of being separated from a medium by means of his physical properties, in particular B comprises CPG, monolithic silica, polymer silica or a polymer material or a monolithic polymer material, wherein in particular B comprises additionally magnetic properties, and
- E comprises a carbon-carbon double bond or a carbon-carbon triple bond, in particular a cyclic double bond, such as for example norbornene or cyclooctene, a tetrazine or a derivative of the aforementioned compounds.

According to a third aspect of the invention provided herein is a compound, particularly a compound for carrying out a process according to the first aspect of the invention, of the general formula 2, 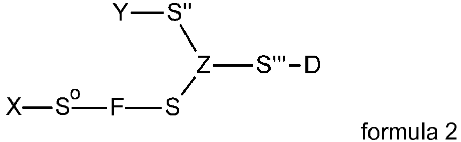 wherein
- X is a reactivity function which can be activated by irradiation and can form after said activation a covalent bond to a target compound T,
- F is a cleavable function,
- Y is a selectivity function selected from the group comprising a bioactive small molecule,
- Z is carbon, nitrogen, phosphorus, silicon, sulfonium, cycloalkyl, unsaturated cycloalkyl, unsaturated heterocycloalkyl or heteroaryl,
- D is a first linking function capable of forming a covalent bond selectively with a second linking function E under reaction conditions not leading to a covalent reaction of D or E with natural occurring polypeptides, in particular with proteins,
- S°, S, S" and S'" are spacer moieties, each of which represents, independently of the others, a bridge between X and F, Z and F, Z and Y or Z and D, facilitated by covalent bonds of carbon, nitrogen, oxygen, sulphur, silicon and/or phosphorous atoms, wherein S°, S, S" and S'" are optional.

According to a fourth aspect of the invention provided herein is a compound, particularly a compound for carrying out a process according to the first aspect of the invention, of the general formula 6, 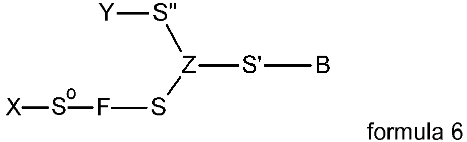 wherein
- X is a reactivity function which can be activated by irradiation and can form after said activation a covalent bond to a target compound T,
- F is a cleavable function,
- Y is a selectivity function selected from the group comprising a bioactive small molecule, in particular
   - Y is bioactive small molecule selected from the group comprising a pharmaceutical drug, a drug development candidate, a drug fragment, a drug metabolite, a prodrug, a herbicide, a fungicide, an insecticide or a natural product, wherein in particular all the before mentioned selectivity functions Y are low-molecular weight compounds, more particularly a pharmaceutical drug, a drug fragment, a drug metabolite, a prodrug, a herbicide or a natural product with less than 1500 u, in particular less than 1000 u, more particularly less than 500 u, in molecular mass,
- Z is carbon, nitrogen, phosphorus, silicon, sulfonium, cycloalkyl, unsaturated cycloalkyl, unsaturated heterocycloalkyl or heteroaryl,
- B is a macroscopic carrier, which is capable of being separated from a medium by means of his physical properties,
- S°, S, S' and S" are spacer moieties, each of which represents, independently of the others, a bridge between X and F, Z and F, Z and B or Z and Y, facilitated by covalent bonds of carbon, nitrogen, oxygen, sulphur, silicon and/or phosphorous atoms, wherein S°, S and S" are optional, and wherein S' comprises the spacer moieties S'" and S^{iv} of formula 2 and 3 and additionally the structural element derived from the reaction of the linking functions D and E.

According to a fifth aspect of the invention provided herein is a compound, particularly a compound for carrying out a process according to the first aspect of the invention, of the general formula 4, 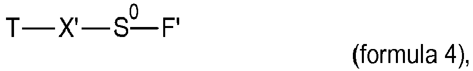 in particular of the general formula 4b or 4b', more particularly 4b', 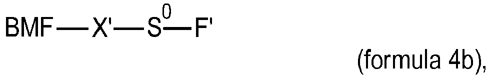 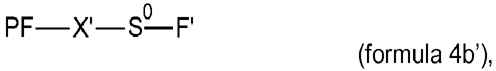 wherein
- X' is the chemical structure resulting from activation of the reactivity function X by irradiation and the subsequent forming of a covalent bond to the target compound T,
- T is a target compound comprising, in particular essentially consisting of, a biomolecule BM or a biomolecule fragment BMF, more particularly a protein P or a protein fragment PF,
- S° is a spacer moiety, which represents a bridge between X' and F', facilitated by covalent bonds of carbon, nitrogen, oxygen, sulphur, silicon and/or phosphorous atoms, wherein S° is optional,
- F' is the chemical structure resulting from a cleavage reaction of a cleavable function F, wherein the cleavable function F is cleavable by H⁺, OH⁻, thiols, salts from acids, fluorides, hydrazides, nucleophiles, electrophiles, radicals, alkylating agents, oxidation, reduction, enzymes or irradiation or a combination of the before mentioned, in particular F is selected from the group comprising an azo-, an acyl-, a disulfide-, a silyoxy-, a carbamate- , an acetal-, a ketal, an arylether, a hydrazone- or an arylketone group.

According to a sixth aspect of the invention provided herein is a combination of reagents for carrying out a process according to the first aspect of the invention, in particular a combination of reagents for carrying out a process according to at least one of the claims 1 to 12, comprising a first compound according to the second aspect of the invention, in particular according to claim 13, and a second compound according to the third aspect of the invention, in particular according to claim 14.

### DETAILED DESCRIPTION OF THE INVENTION

According to the first aspect, the invention relates to a process for isolating and/or characterising biomolecules and/or biomolecule fragments, in particular proteins and/or protein fragments, comprising the steps:
a. provision of an immobilized compound IC of the general formula 1 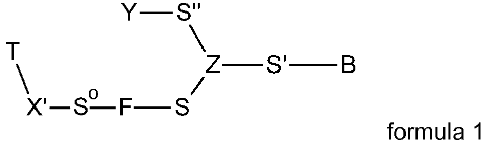 wherein
   - T is a target compound comprising, in particular essentially consisting of, a biomolecule BM or a biomolecule fragment BMF, more particularly a protein P or a protein fragment PF,
   - X' is the chemical structure resulting from activation of the reactivity function X by irradiation and the subsequent forming of a covalent bond to the target compound T,
   - F is a cleavable function,
   - Y is a selectivity function selected from the group comprising a bioactive small molecule,
   - Z is carbon, nitrogen, phosphorus, silicon, sulfonium, cycloalkyl, unsaturated cycloalkyl, unsaturated heterocycloalkyl or heteroaryl,
   - B is a macroscopic carrier, which is capable of being separated from a medium by means of his physical properties,
   - S°, S, S' and S" are spacer moieties, each of which represents, independently of the others, a bridge between X' and F, Z and F, Z and B or Z and Y, facilitated by covalent bonds of carbon, nitrogen, oxygen, sulphur, silicon and/or phosphorous atoms, wherein S°, S and S" are optional, and wherein S' comprises the spacer moieties S'" and S'" of formula 2 and 3 and additionally the structural element derived from the reaction of the linking functions D and E;
      through the interaction of
      i. a capture compound CC of the general formula 2 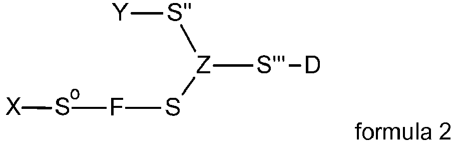 wherein
         - X is a reactivity function which can be activated by irradiation and can form after said activation a covalent bond to the target compound T, and
         - F, Y, Z, S, S° and S" each have the meanings defined previously, and
         - S'" is a spacer moiety representing a bridge between Z and D, facilitated by covalent bonds of carbon, nitrogen, oxygen, sulphur, silicon and/or phosphorous atoms, wherein S'" is optional, and
         - D is a first linking function capable of forming a covalent bond selectively with a second linking function E under reaction conditions not leading to a covalent reaction of D or E with natural occurring polypeptides, in particular with proteins;
      ii. the target compound T; and
      iii. a carrier compound CCB of the general formula 3 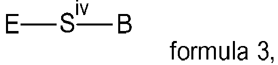 wherein
         - S^{iV} is a spacer moiety representing a bridge between E and B facilitated by covalent bonds of carbon, nitrogen, oxygen, sulphur, silicon and/or phosphorous atoms, wherein S^{iv} is optional,
         - B has the meaning defined previously, and
         - E is said second linking function; and
b. carrying out a division step, in which the cleavable function F is cleaved, and a division compound DC of the general formula 4 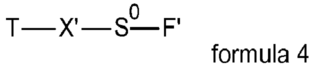 is produced, wherein
   - T, S⁰ and X' have the meanings defined previously, wherein S° is optional, and
   - F' is the chemical structure resulting from a cleavage reaction of the cleavable function F; and
c. isolation and/or characterisation of said division compound DC.

In some embodiments, the provision of an immobilized compound ICa, wherein T is at least one biomolecule BM or at least one protein P, is achieved by means of the following steps:
a. carrying out a bonding step, wherein a biological sample comprising one or more biomolecules, in particular one or more proteins is brought into contact with the capture compound CC of formula 2 under conditions leading to a selective interaction of said selectivity function Y with at least one biomolecule BM or at least one protein P of the biological sample, and subsequently
b. an activation of the reactivity function X by the effect of irradiation is carried out, wherein subsequently said reactivity function X forms a covalent bond with BM or P yielding the activated function X', producing a protein capture compound PCC of the general formula 5 or 5', in particular 5', 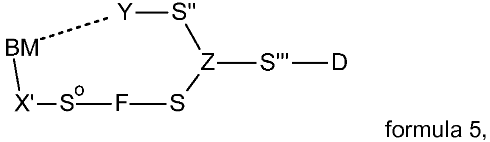 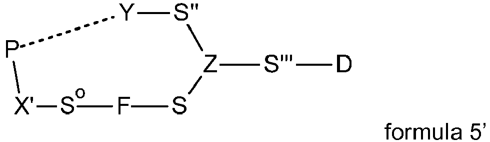 wherein X', Z, P, BM, S^{o}, S, S", S"', Y, D and F have the meanings defined previously; and subsequently
c. carrying out a further bonding step, wherein the protein capture compound PCC of formula 5 or 5' is brought into contact with the carrier compound CCB of formula 3 under conditions resulting in a covalent bond between D and E, and producing an immobilized compound ICa of the general formula 1a or 1a', in particular 1a', 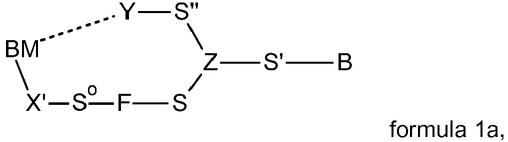 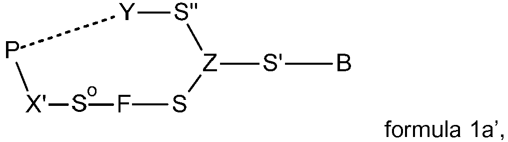 wherein X', Z, P, BM, S^{o}, S, S", Y, B and F have the meanings given previously, and wherein S' comprises the spacer moieties S'" and S^{iv} of formula 3 and 5 or 5' and additionally the structural element derived from the reaction of the linking functions D and E; and subsequently
d. isolation of the immobilized compound ICa of formula 1 a or 1a'.

In some embodiments, the provision of an immobilized compound ICa, wherein T is at least one biomolecule BM or at least one protein P, is achieved by means of the following steps:
a. carrying out a bonding step, wherein the carrier compound CCB of formula 3 is brought into contact with the capture compound CC of formula 2 under conditions resulting in a covalent bond between D and E, producing a precursor compound PC of the general formula 6 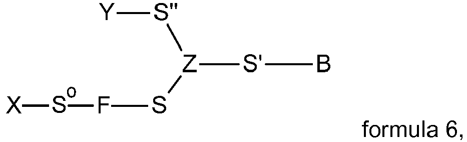 wherein X, Z, S°, S, S', S", Y, B and F have the meanings given previously; and subsequently
b. carrying out a further bonding step, wherein a biological sample comprising one or more proteins is brought into contact with the precursor compound PC of formula 6 under conditions leading to a selective interaction of said selectivity function Y with at least one biomolecule BM or at least one protein P from the biological sample; and subsequently
c. an activation of the reactivity function X by the effect of irradiation is carried out, wherein subsequently said reactivity function X forms a covalent bond with BM or P yielding the function X', producing the immobilized compound ICa of formula 1a or 1 a',
   wherein X', Z, P,BM, S^{o}, S, S', S", Y, B and F have the meanings given previously; and subsequently
d. isolation of the immobilized compound ICa of formula 1a or 1a'.

In some embodiments, a compound of the formula 1 a or 1 a' is provided, in particular according to one of the previously discussed steps, and
a. before the division step b of the previous discussed embodiments, the at least one biomolecule BM or the at least one protein P of the isolated compound of formula 1a or 1a' is broken down into individual fragment parts in a digestive step, producing an immobilized compound ICb of the general formula 1b or 1b', in particular 1b', 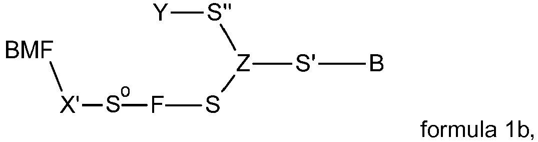 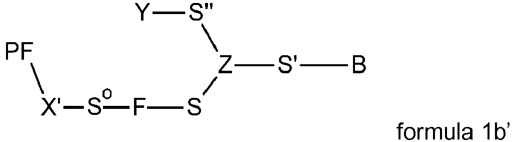 wherein
   - X', Z, PF, BMF, S^{o}, S, S', S", Y, B and F have the meanings defined previously, and
   - BMF or PF are the fragment parts of the at least one digested biomolecule BM or the at least one digested protein P which are bound to the immobilising compound ICb of formula 1b or 1b' by the function X'; and subsequently
b. a division step is carried out, in which the cleavable function F of the compound of formula 1 b or 1 b' is cleaved, producing a division compound DCb of the general formula 4b or 4b', in particular 4b', 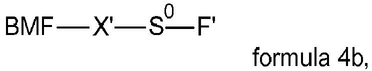 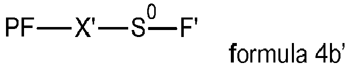 wherein BMF, PF, S⁰ and X' have the meanings defined previously, and F' is the chemical structure resulting from a cleavage reaction of the cleavable function F; and subsequently
c. an isolation and/or a characterisation step of said division compound DCb of formula 4b or 4b' is carried out or a characterisation step of said division compound DCb of formula 4b or 4b' and the further fragment parts of the at least one digested biomolecule BM or the at least one digested protein P, which are not bound to the immobilising compound ICb of formula 1 b or 1 b', is carried out.

In some embodiments, a compound of the formula 1 a or 1 a' is provided, in particular according to one of the previously discussed steps, and a division step is carried out, in which the cleavable function F is cleaved, producing a division compound DCa of the general formula 4a or 4a', in particular 4a', 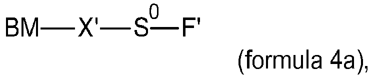 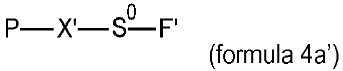 wherein F', S° and X have the meanings defined previously, and BM is the biomolecule or P is the protein which is bound to the division compound DCa of formula 4a or 4a' by the function X', and subsequently an isolation and/or a characterisation step of said division compound DCa of formula 4a or 4a' is carried out.

In some embodiments, a compound of the formula 1a or 1a' is provided, in particular according to one of the previously discussed steps, and a division step is carried out, in which the cleavable function F is cleaved, producing a division compound DCa of the general formula 4a or 4a', and subsequently the at least one biomolecule BM or the at least one protein P of the compound of the formula 4a or 4a' is broken down into individual fragment parts in a digestive step, producing a division compound DCb of the general formula 4b or 4b', in particular 4b, 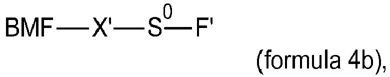 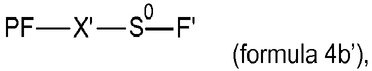 and subsequently
an isolation and/or a characterisation step of said division compound DCb of formula 4b or 4b' is carried out, wherein F', S⁰ and X' have the meanings defined previously, and BMF and PF are the fragment parts of the at least one digested biomolecule BM or the at least one digested protein P which are bound to the division compound DCb of formula 4b or 4b' by the function X' or a characterisation step of said division compound DCb of formula 4b or 4b' and the further fragment parts of the at least one digested biomolecule BM or the at least one digested protein P, which are not bound to the immobilising compound ICb of formula 1 b or 1 b', is carried out.

In some embodiments, the process for isolating and/or characterising biomolecules and/or biomolecule fragments, in particular proteins and/or protein fragments comprises
a. the provision of an immobilized compound ICa of the general formula 1 a or 1 a' by means of the following steps:
   - carrying out a bonding step, wherein a biological sample comprising one or more biomolecules, in particular one or more proteins is brought into contact with the capture compound CC of formula 2 under conditions leading to a selective interaction of said selectivity function Y with at least one biomolecule BM or at least one protein P of the biological sample, and subsequently
   - an activation of the reactivity function X by the effect of irradiation is carried out, wherein subsequently said reactivity function X forms a covalent bond with BM or P yielding the function X', producing a protein capture compound PCC of the general formula 5 or 5', and subsequently
   - carrying out a further bonding step, wherein the protein capture compound PCC of formula 5 or 5' is brought into contact with the carrier compound CCB of formula 3 under conditions resulting in a covalent bond between D and E, producing the immobilized compound ICa of the general formula 1a or 1a', and subsequently
   - isolation of the immobilized compound ICa of formula 1a or 1a'; and subsequently
b. a digestive step is applied to the isolated immobilized compound ICa of formula 1 a or 1 a', wherein the at least one biomolecule BM or the at least one protein P of the compound of formula 1 a or 1 a' is broken down into individual fragment parts, producing an immobilized compound ICb of the general formula 1 b or 1 b', and subsequently
c. a division step is carried out, in which the cleavable function F of the compound of formula 1 b or 1 b' is cleaved, producing a division compound DCb of the general formula 4b or 4b', and subsequently
d. an isolation and/or a characterisation step of said division compound DCb of formula 4b or 4b' is carried out or a characterisation step of said division compound DCb of formula 4b or 4b'and the further fragment parts of the at least one digested biomolecule BM or the at least one digested protein P, which are not bound to the immobilising compound ICb of formula 1 b or 1 b', is carried out.

The steps of the previous discussed process are depicted in scheme 1.

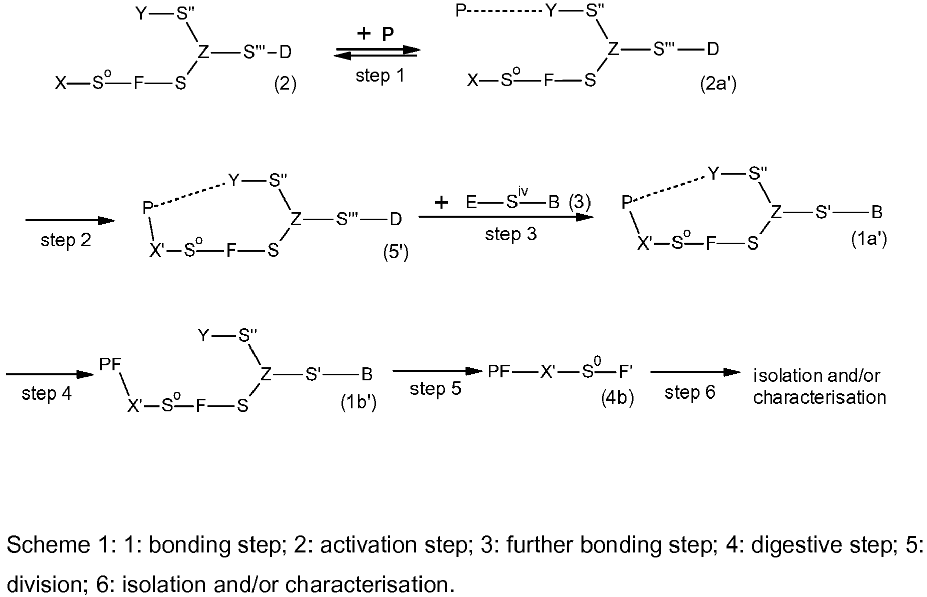

In some embodiments, the process for isolating and/or characterising biomolecules and/or biomolecule fragments, in particular proteins and/or protein fragments comprises
a. the provision of an immobilized compound ICa of the general formula 1a or 1a' by means of the following steps:
   - carrying out a bonding step, wherein a biological sample comprising one or more biomolecules, in particular one or more proteins, is brought into contact with the capture compound CC of formula 2 under conditions leading to a selective interaction of said selectivity function Y with at least one biomolecule BM or at least one protein P of the biological sample, and subsequently
   - an activation of the reactivity function X by the effect of irradiation is carried out, wherein subsequently said reactivity function X forms a covalent bond with BM or P yielding the function X', producing a protein capture compound PCC of the general formula 5 or 5', and subsequently
   - carrying out a further bonding step, wherein the protein capture compound PCC of formula 5 or 5' is brought into contact with the carrier compound CCB of formula 3 under conditions resulting in a covalent bond between D and E, producing the immobilized compound ICa of the general formula 1a or 1a', and subsequently
   - isolation of the immobilized compound ICa of formula 1a or 1a'; and subsequently
b. a digestive step is applied to the isolated immobilized compound ICa of formula 1a or 1a', wherein the at least one biomolecule BM or the at least one protein P of the compound of formula 1 a or 1 a' is broken down into individual fragment parts, producing an immobilized compound ICb of the general formula 1 b or 1 b', and subsequently
c. isolation of the immobilized compound ICb of the general formula 1 b or 1 b', and subsequently
d. a division step is carried out, in which the cleavable function F of the compound of formula 1 b or 1 b' is cleaved, producing a division compound DCb of the general formula 4b or 4b', and subsequently
e. an isolation and/or a characterisation step of said division compound DCb of formula 4b or 4b' is carried out or a characterisation step of said division compound DCb of formula 4b or 4b' and the further fragment parts of the at least one digested biomolecule BM or the at least one digested protein P, which are not bound to the immobilising compound ICb of formula 1 b or 1 b', is carried out.

The steps of the previous discussed process are depicted in scheme 2.

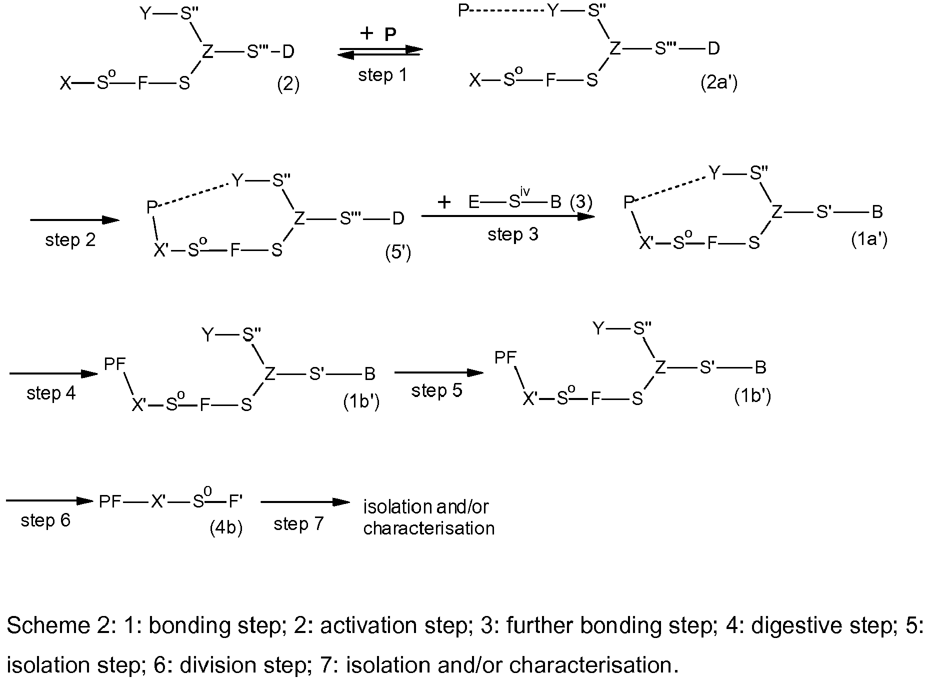

In some embodiments, the process for isolating and/or characterising biomolecules and/or biomolecule fragments, in particular proteins and/or protein fragments comprises
a. the provision of an immobilized compound ICa of the general formula 1 a or 1 a' by means of the following steps:
   - carrying out a bonding step, wherein a biological sample comprising one or more biomolecules, in particular one or more proteins, is brought into contact with the capture compound CC of formula 2 under conditions leading to a selective interaction of said selectivity function Y with at least one biomolecule BM or at least one protein P of the biological sample, and subsequently
   - an activation of the reactivity function X by the effect of irradiation is carried out, wherein subsequently said reactivity function X forms a covalent bond with BM or P yielding the function X', producing a protein capture compound PCC of the general formula 5 or 5', and subsequently
   - carrying out a further bonding step, wherein the protein capture compound PCC of formula 5 or 5' is brought into contact with the carrier compound CCB of formula 3 under conditions resulting in a covalent bond between D and E, producing the immobilized compound ICa of the general formula 1a or 1a', and subsequently
   - isolation of the immobilized compound ICa of formula 1a or 1a'; and subsequently
b. a division step is carried out, in which the cleavable function F of the compound of formula 1 a or 1 a' is cleaved, producing a division compound DCa of the general formula 4a or 4a', and subsequently
c. an isolation and/or a characterisation step of said division compound DCa of formula 4a or 4a' is carried out.

In some embodiments, the process for isolating and/or characterising biomolecules and/or biomolecule fragments, in particular proteins and/or protein fragments comprises
a. the provision of an immobilized compound ICa of the general formula 1 a or 1 a' by means of the following steps:
   - carrying out a bonding step, wherein a biological sample comprising one or more biomolecules, in particular one or more proteins, is brought into contact with the capture compound CC of formula 2 under conditions leading to a selective interaction of said selectivity function Y with at least one biomolecule BM or at least one protein P of the biological sample, and subsequently
   - an activation of the reactivity function X by the effect of irradiation is carried out, wherein subsequently said reactivity function X forms a covalent bond with BM or P yielding the function X', producing a protein capture compound PCC of the general formula 5 or 5', and subsequently
   - carrying out a further bonding step, wherein the protein capture compound PCC of formula 5 or 5' is brought into contact with the carrier compound CCB of formula 3 under conditions resulting in a covalent bond between D and E, producing the immobilized compound ICa of the general formula 1a or 1a', and subsequently
   - isolation of the immobilized compound ICa of formula 1a or 1a'; and subsequently
b. a division step is carried out, in which the cleavable function F of the compound of formula 1 a or 1 a' is cleaved, producing a division compound DCa of the general formula 4a or 4a', which is isolated, and subsequently
c. a digestive step is applied to the isolated division compound DCa of the general formula 4a or 4a', wherein the at least one biomolecule BM or the at least one protein P of the compound of formula 4a or 4a' is broken down into individual fragment parts, producing a division compound DCb of the general formula 4b or 4b', and subsequently
d. an isolation and/or a characterisation step of said division compound DCb of formula 4b or 4b' is carried out or a characterisation step of said division compound DCb of formula 4b or 4b' and the further fragment parts of the at least one digested biomolecule BM or at least one digested protein P, which are not bound to the immobilising compound ICb of formula 1 b or 1 b', is carried out.

The steps of the previous discussed process are depicted in scheme 3.

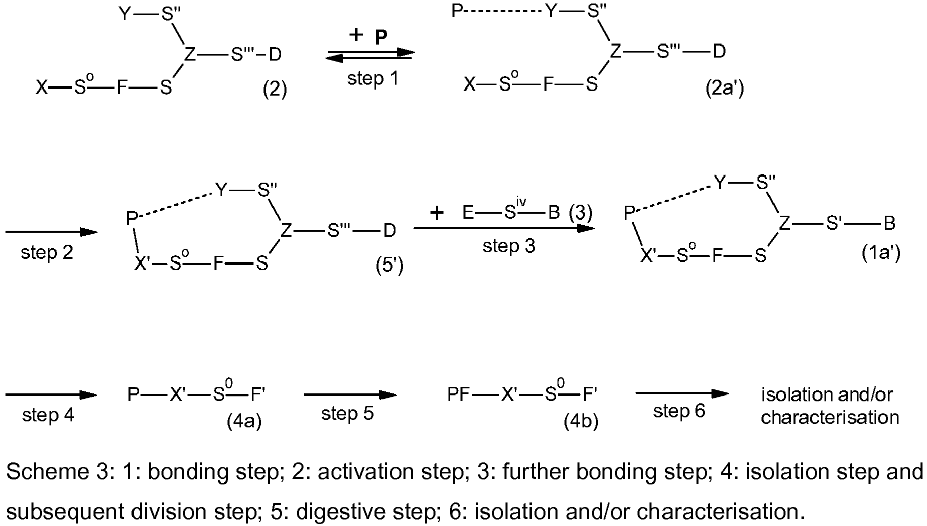

In some embodiments, the process for isolating and/or characterising biomolecules and/or biomolecule fragments, in particular proteins and/or protein fragments comprises
a. the provision of an immobilized compound ICa of the general formula 1 a or 1 a' by means of the following steps:
   - carrying out a bonding step, wherein the carrier compound CCB of formula 3 is brought into contact with the capture compound CC of formula 2 under conditions resulting in a covalent bond between D and E, producing a precursor compound PC of the general formula 6, and subsequently
   - carrying out a further bonding step, wherein a biological sample comprising one or more biomolecules, in particular one or more proteins, is brought into contact with the precursor compound PC of formula 6 under conditions leading to a selective interaction of said selectivity function Y with at least one biomolecule BM or at least one protein P from the biological sample; and subsequently
   - an activation of the reactivity function X by the effect of irradiation is carried out, wherein subsequently said reactivity function X forms a covalent bond with BM or P yielding the function X', producing the immobilized compound ICa of formula 1a or 1a', and subsequently
   - isolation of the immobilized compound ICa of formula 1a or 1a'; and subsequently
b. a digestive step is applied to the isolated immobilized compound ICa of formula 1 a or 1a', wherein the at least one biomolecule BM or the at least one protein P of the compound of formula 1 a or 1 a' is broken down into individual fragment parts, producing an immobilized compound ICb of the general formula 1 b or 1 b', and subsequently
c. a division step is carried out, in which the cleavable function F of the compound of formula 1 b or 1 b' is cleaved, producing a division compound DCb of the general formula 4b or 4b', and subsequently;
d. an isolation and/or a characterisation step of said division compound DCb of formula 4b or 4b' is carried out or a characterisation step of said division compound DCb of formula 4b or 4b'and the further fragment parts of at least one digested biomolecule BM or the at least one digested protein P, which are not bound to the immobilising compound ICb of formula 1 b or 1 b', is carried out.

The steps of the previous discussed process are depicted in scheme 4.

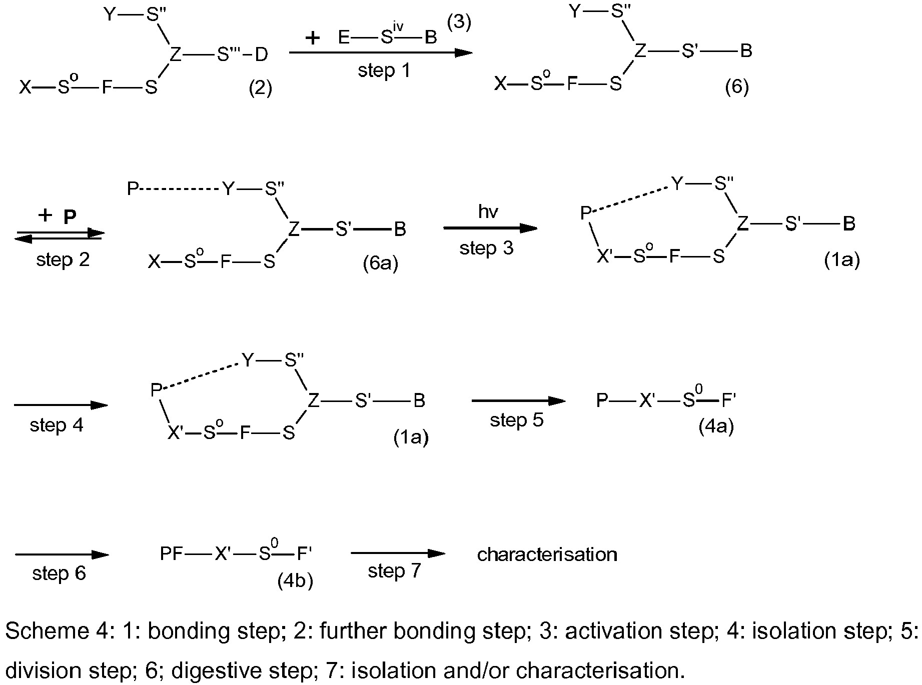

In some embodiments, the process for isolating and/or characterising biomolecules and/or biomolecule fragments, in particular proteins and/or protein fragments comprises
a. the provision of an immobilized compound ICa of the general formula 1a or 1a' by means of the following steps:
   - carrying out a bonding step, wherein the carrier compound CCB of formula 3 is brought into contact with the capture compound CC of formula 2 under conditions resulting in a covalent bond between D and E, producing a precursor compound PC of the general formula 6, and subsequently
   - carrying out a further bonding step, wherein a biological sample comprising one or more biomolecules, in particular one or more proteins, is brought into contact with the precursor compound PC of formula 6 under conditions leading to a selective interaction of said selectivity function Y with at least one biomolecule BM or at least one protein P from the biological sample; and subsequently
   - an activation of the reactivity function X by the effect of irradiation is carried out, wherein subsequently said reactivity function X forms a covalent bond with BM or P yielding the function X', producing the immobilized compound ICa of formula 1a or 1a', and subsequently
   - isolation of the immobilized compound ICa of formula 1a or 1a'; and subsequently
b. a digestive step is applied to the isolated immobilized compound ICa of formula 1 a or 1 a', wherein the at least one biomolecule BM or the at least one protein P of the compound of formula 1 a or 1 a' is broken down into individual fragment parts, producing an immobilized compound ICb of the general formula 1 b or 1 b', and subsequently
c. isolation of the immobilized compound ICb of the general formula 1 b or 1 b', and subsequently
d. a division step is carried out, in which the cleavable function F of the compound of formula 1 b or 1 b' is cleaved, producing a division compound DCb of the general formula 4b or 4b', and subsequently
e. an isolation and/or a characterisation step of said division compound DCb of formula 4b or 4b' is carried out.

The steps of the previous discussed process are depicted in scheme 5.

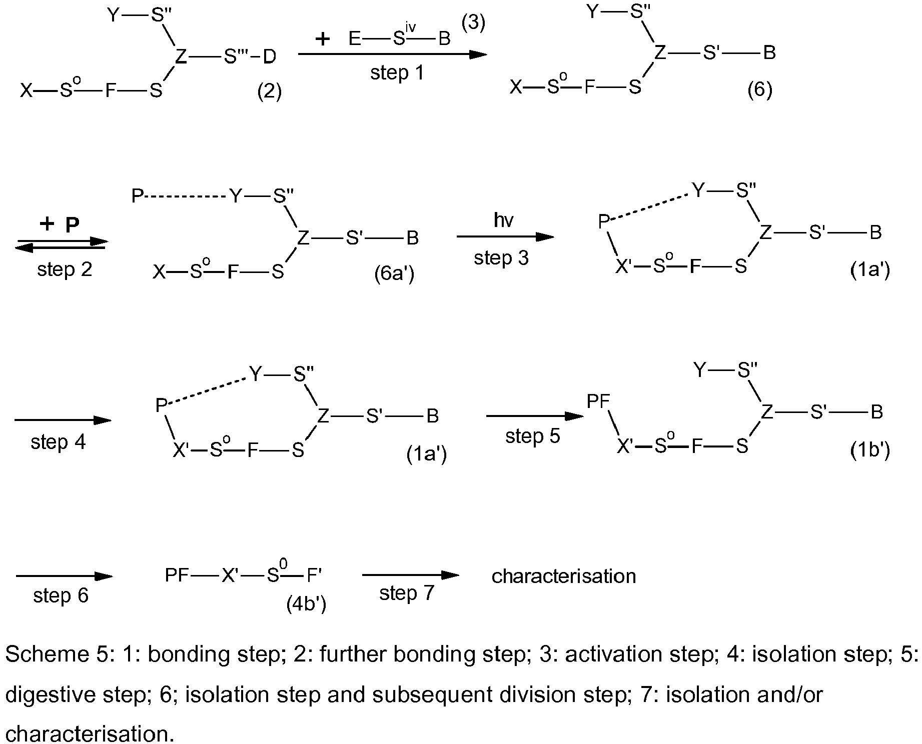

In some embodiments, the process for isolating and/or characterising biomolecules and/or biomolecule fragments, in particular proteins and/or protein fragments comprises
a. the provision of an immobilized compound ICa of the general formula 1 a or 1 a' by means of the following steps:
   - carrying out a bonding step, wherein the carrier compound CCB of formula 3 is brought into contact with the capture compound CC of formula 2 under conditions resulting in a covalent bond between D and E, producing a precursor compound PC of the general formula 6, and subsequently
   - carrying out a further bonding step, wherein a biological sample comprising one or more biomolecules, in particular one or more proteins, is brought into contact with the precursor compound PC of formula 6 under conditions leading to a selective interaction of said selectivity function Y with at least one biomolecule BM or at least one protein P from the biological sample; and subsequently
   - an activation of the reactivity function X by the effect of irradiation is carried out, wherein subsequently said reactivity function X forms a covalent bond with BM or P yielding the function X', producing the immobilized compound ICa of formula 1a or 1a', and subsequently
   - isolation of the immobilized compound ICa of formula 1a or 1a'; and subsequently
b. a division step is carried out, in which the cleavable function F of the compound of formula 1 a or 1 a' is cleaved, producing a division compound DCa of the general formula 4a or 4a', and subsequently
c. an isolation and/or a characterisation step of said division compound DCa of formula 4a or 4a' is carried out.

In some embodiments, the process for isolating and/or characterising biomolecules and/or biomolecule fragments, in particular proteins and/or protein fragments comprises
a. the provision of an immobilized compound ICa of the general formula 1 a or 1 a' by means of the following steps:
   - carrying out a bonding step, wherein the carrier compound CCB of formula 3 is brought into contact with the capture compound CC of formula 2 under conditions resulting in a covalent bond between D and E, producing a precursor compound PC of the general formula 6, and subsequently
   - carrying out a further bonding step, wherein a biological sample comprising one or more biomolecules, in particular one or more proteins, is brought into contact with the precursor compound PC of formula 6 under conditions leading to a selective interaction of said selectivity function Y with at least one biomolecule BM or at least one protein P from the biological sample; and subsequently
   - an activation of the reactivity function X by the effect of irradiation is carried out, wherein subsequently said reactivity function X forms a covalent bond with BM or P yielding the function X', producing the immobilized compound ICa of formula 1a or 1a', and subsequently
   - isolation of the immobilized compound ICa of formula 1a or 1a'; and subsequently
e. a division step is carried out, in which the cleavable function F of the compound of formula 1 a or 1 a' is cleaved, producing a division compound DCa of the general formula 4a or 4a', which is isolated, and subsequently
b. a digestive step is applied to the isolated division compound DCa of the general formula 4a or 4a', wherein the at least one biomolecule BM or the at least one protein P of the compound of formula 4a or 4a' is broken down into individual fragment parts, producing a division compound DCb of the general formula 4b or 4b', and subsequently
c. an isolation and/or a characterisation step of said division compound DCb of formula 4b or 4b' is carried out or a characterisation step of said division compound DCb of formula 4b or 4b' and the further fragment parts of the at least one digested biomolecule BM or the at least one digested protein P, which are not bound to the immobilising compound ICb of formula 1 b or 1 b', is carried out.

In some embodiments, an immobilized compound ICb of general formula 1 b or 1 b' is provided by means of the following steps:
a. carrying out a bonding step, wherein a biological sample comprising one or more biomolecules, in particular one or more proteins, is brought into contact with the capture compound CC of formula 2 under conditions leading to the selective interaction of said selectivity function Y with the at least one biomolecule BM or the at least one protein P of the biological sample; and subsequently
b. an activation of said reactivity function X by the effect of irradiation is carried out, wherein subsequently said reactivity function X forms a covalent bond with BM or P producing a protein capture compound PCC of the general formula 5 or 5', in particular 5' 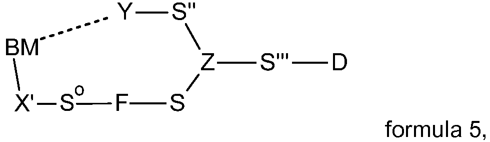 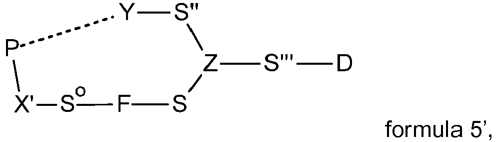 and subsequently
c. said at least one biomolecule BM or at least one protein P of the compound of formula 5 or 5' is broken down into individual fragment parts in a digestive step producing a protein fragment capture compound PFCC of the general formula 5a or 5a', in particular 5a', 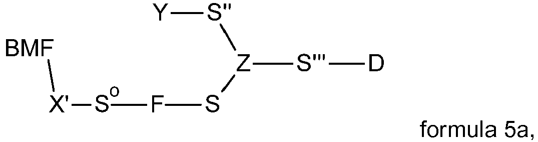 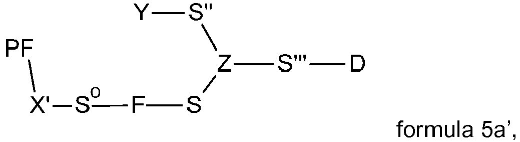 and subsequently
d. carrying out of a further bonding step, wherein the protein fragment capture compound PFCC of formula 5a or 5a' is brought into contact with the carrier compound CCB of formula 3 under conditions resulting in a covalent bond between D and E producing the immobilising compound ICb of formula 1b or 1b', and subsequently
e. isolation of the immobilising compound ICb of the general formula 1b or 1b'.

In some embodiments, the process for isolating and/or characterising biomolecules and/or biomolecule fragments, in particular proteins and/or protein fragments comprises
a. the provision of an immobilized compound ICb of the general formula 1 b or 1 b' by means of the following steps:
   - carrying out a bonding step, wherein a biological sample comprising one or more biomolecules, in particular one or more proteins, is brought into contact with the capture compound CC of formula 2 under conditions leading to the selective interaction of said selectivity function Y with the at least one biomolecule BM or the at least one protein P of the biological sample; and subsequently
   - an activation of said reactivity function X by the effect of irradiation is carried out, wherein subsequently said reactivity function X forms a covalent bond with BM or P producing a protein capture compound PCC of the general formula 5 or 5', and subsequently
   - a digestive step is carried out, wherein said at least one biomolecule BM or at least one protein P of the compound of formula 5 or 5' is broken down into individual fragment parts producing a protein fragment capture compound PFCC of the general formula 5a or 5a', and subsequently
   - carrying out of a further bonding step, wherein the protein fragment capture compound PFCC of formula 5a or 5a' is brought into contact with the carrier compound CCB of formula 3 under conditions resulting in a covalent bond between D and E producing the immobilising compound ICb of formula 1 b or 1 b', and subsequently
   - isolation of the immobilising compound ICb of the general formula 1 b or 1 b', and subsequently
b. a division step is carried out, in which the cleavable function F of the compound of formula 1 b or 1 b' is cleaved, producing a division compound DCb of the general formula 4b or 4b', and subsequently
c. an isolation and/or a characterisation step of said division compound (DC) of formula 4b or 4b' is carried out.

The steps of the previous discussed process are depicted in scheme 6.

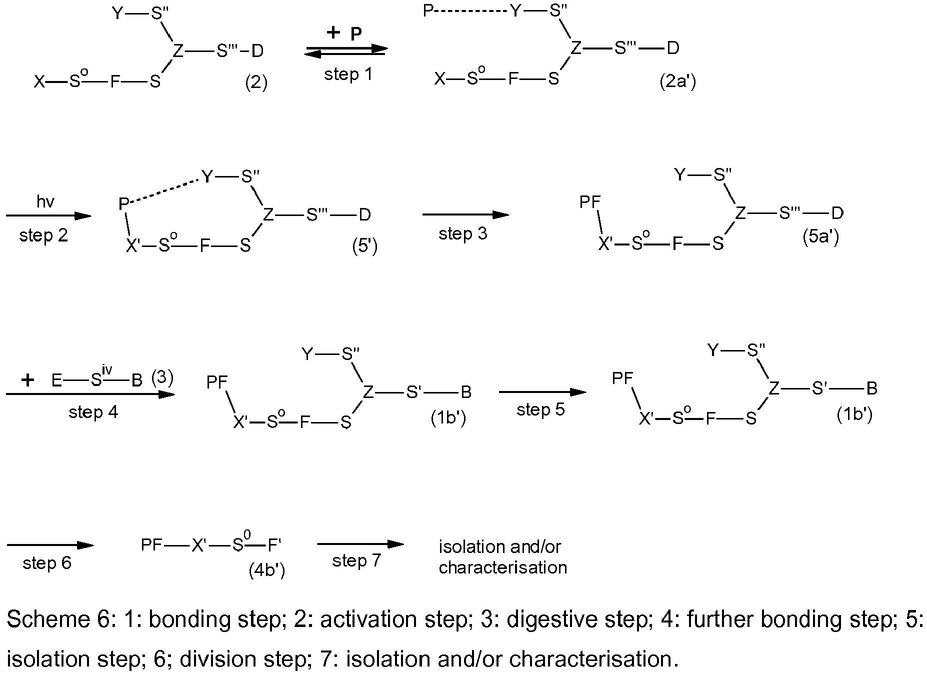

If not stated otherwise, any of the embodiments described below can be combined with all of the above mentioned embodiments of the process according to the invention.

In some embodiments, the target compound comprises, in particular essentially consist of, a biomolecule BM or a biomolecule fragment BMF, more particularly a protein P or a protein fragment PF selected from the group of membrane, fibrous and/or globular proteins, in particular from the group of membrane proteins and cytosolic proteins or proteins derived from subcellular fractions such as nuclei or mitochondria.

The selectivity function Y comprises an affinity to the (non/off-) target protein(s) P (or a biomolecule BM). The affinity is characterized by a dissociation constant below 10 mmol/l. In some embodiments, the dissociation constant is below 1 mmol/l. In some embodiments, the dissociation constant is below 100 µmol/l. In some embodiments, the dissociation constant is below 10 µmol/l. In some embodiments, the dissociation constant is below 1 µmol/m. In some embodiments, the dissociation constant is in the nanomolar range. In some embodiments, the dissociation constant is below <100 nmol/l.

In some embodiments, the reactivity function X is a moiety that can be stimulated to generate a reactive species (activation of the reactivity function) which will quickly form a covalent bond with a range of suitable partners of a target compound. Thus, the reactivity function X forms a covalent bond irreversibly linking the compound comprising the reactivity function X to those target compounds for which there was an affinity.

In some embodiments, the reactivity function X can be activated by exposure to light comprising a wavelength λₘₐₓ in the range of 250 nm to 400 nm, in particular a wavelength λ of 300 nm to 360 nm. After the activation target compounds (proteins) in close proximity react with the highly reactive species formed by activation of X and form a covalent bond between the target compound T and the compound comprising the reactivity function X. Thus, the activation of the reactivity function X and the subsequent reaction with the target compound T yields a function X', derived from said reaction, which covalently connects the compound comprising the reactivity function X and the target compound T. In other words, the target compound is now "captured" through a covalent crosslink.

One example for such light source is the "caprobox" (caprotec bioanalytics GmbH, Berlin). Another example is a common trans-illuminator for DNA agarose gel analysis, which generates light of ca. 360 nm and 254 nm. Further examples are the stratalinker (Stratagene/Agilent), which can generate light at different UV wavelengths or a lamp from AlienBees Self-Contained Studio Flash Lamp.

Non-limiting examples for activation reactions for said reactivity functions X are the generation of a carbene by photolysis of a diazo compound, for example a diazirine, or the generation of a nitrene from an azide, or the generation of a (di-)radical from a carbon-hetero atom double bond, any of which reactive intermediate species will quickly go on to react further with -by way of non-limiting example- an aromatic amino acid side chain, or an alcohol-, amino- or thiole function or amide bond of a sterically proximate amino acid of the polypeptide chain.

In some embodiments, X is selected from a diazirine-, an arylazide-, a di- or tetrahalogenarylazide or an arylketone group, wherein X is selected particularly from the group comprising aryltrifluoromethyldiazirine or tetrahalogenarylazide. Aryl azides may comprise one or two azides group on the aromatic ring.

Non-limiting examples for reactivity functions X are: 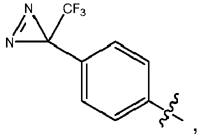 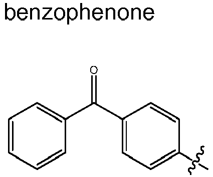 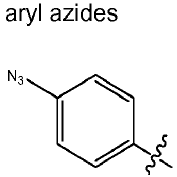 or

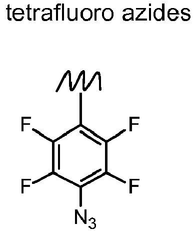

The covalent link of the reactivity function X to the target compound T can be realized through another than the indicated positions, for example through position 2 or 3 in the ring. The aromatic rings may be substituted by, for example, cyano groups, nitro groups or hydroxyl groups.

The reactivity function X, as discussed above, forms upon activation a covalent bond to any protein in its proximity. In absence of a selectivity function Y the capture compound would capture randomly a more or less representative selection of any proteins present in the sample. The selectivity function restricts the linkage of the capture compound to such proteins that specifically interact with the selectivity function Y based on affinity, as discussed below.

The selectivity function Y selects through a non-covalent interaction the types of target compounds that can interact based on affinity .Thereafter the reactivity function X is activated to freeze-in the equilibrium. The result is a functionally enriched sub-proteome based on the functional relationship between T and Y. The selectivity function Y ,generally reduces the number of target compounds T covalently connected ("captured") to an immobilized compound IC, as described above, so that the target compounds T can then be isolated and/or characterized, for example by mass spectrometry.

The selectivity function Y in principle may be any molecular moiety with highly selective affinity to target (off-target-) proteins in the micromolar, nanomolar or sub-nanomolar range. Selective interaction of Y with a target protein will restrict the covalent linkage of the reactivity function X to certain proteins. The term "target" protein in this context is somewhat arbitrary. When used in the context of pharmaceutical drug development, the term "target" protein is used to designate the protein that the drug is meant to interact with, or assumed to interact with, thus forming the desired target-drug interaction necessary for the therapeutic action. Proteins which interact with the drug molecule that are not involved in the mode of therapeutic action are defined as "non-target" or "off-target" protein, which however may turn out to be important for the leading to adverse side effects or protein targets for secondary medical indications. The selectivity function Y comprises a bioactive small molecule and is allowed to be extensively varied depending on the goal to be achieved.

In some embodiment, the selectivity function Y is a chiral group, which allows for stereoselective capture of target compounds T.

One important field of application for the instant invention is drug development, in particular the analysis of drug-protein interactions and the improvement of specificity and selectivity for the (desired) drug-target interaction, and likewise the modification of (in most cases, undesired) drug-non-target interactions. For embodiments addressing such applications, Y is a pharmaceutical drug, a drug development candidate, a drug fragment, a drug metabolite or a prodrug, wherein all of the before mentioned can be characterized as small molecules. Other important exemplary applications are herbicides, insecticides, pesticides, fungicides or other small organic bioactive molecules of relevance in any biomedical context. In other embodiments, Y is a pharmaceutically active natural product.

In some embodiments, the selectivity function Y is a bioactive small molecule, as discussed above, of a molecular mass of less than 1500 u. In one embodiment, the selectivity function Y is a bioactive small molecule, as discussed above, of a molecular mass of less than 1000 u. In one embodiment, the selectivity function Y is a bioactive small molecule, as discussed above, of a molecular mass of less than 500 u.

In some embodiment, Y is a molecular moiety obeying the "Lipinski Rule of Five", i.e. Y has a molecular mass between 160 u and 500u, comprises up to five hydrogen bond donators (e.g. oxygen and or nitrogen atoms with one H attached), up to ten hydrogen bond acceptors (e.g., oxygen or nitrogen atoms) and an octanol-water partition coefficient logP of below 5,6 (any of these characteristics applied to the isolated Y moiety, without regard to the remaining moieties of the respective capture compounds).

Binding of the selectivity function Y to the (on/off-) target protein(s) is effected by non-covalent interaction. The affinity of the bioactive molecule, in particular the bioactive small molecule, that corresponds to the selectivity function Y (measured with no further compound attached) is characterized by a dissociation constant in the micromolar, nanomolar or sub-nanomolar range.

The cleavable function F refers to a bond or moiety (also referred to as a "biocompatible cleaving site") that is cleaved or cleavable under the specific conditions, such as chemically, enzymatically or by irradiation thereby releasing a portion (target site) from the compound IC of the formula 1 including the target compound T.

As used herein, a "cleavable function" F is a moiety that can be selectively cleaved without affecting or altering the composition of the other portions of the compound of interest or a cleavage of any other moiety of the immobilized compound IC of the formula 1. For example, a cleavable function F of the compounds provided herein is one that can be cleaved by chemical, enzymatic, photolytic, or other means without affecting or altering the composition (e. g. the chemical composition) of the covalently connected target molecule or a cleavage of any other moiety of the immobilized compound IC of the formula 1.

The conditions for a cleavage of the cleavable function F depends on the chosen cleaving function. The respective cleavable function - depending on the target compound and the remaining moieties of the immobilized compound IC - can be chosen from different groups.

In some embodiments, F is a cleavable function, which is cleavable by H⁺, OH⁻, thiols, salts from acids, fluorides, hydrazides, nucleophiles, electrophiles, radicals, alkylating agents, oxidation, reduction, enzymes or irradiation or a combination of the before mentioned.

In some embodiments, F is selected from the group comprising an azo-, an acyl-, a disulfide-, a silyoxy-, a carbamate- , an acetal-, a ketal, an arylether, a hydrazone-, an arylketone, or an o-nitrobenzyl group.

### Protecting group

### organi

In some embodiments, a cleavable function F, is selected from
- an azo group (cleavage conditions: reductive) 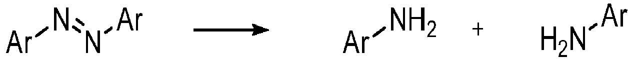
- a thio group (cleavage conditions: reductive) 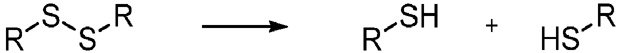
- a carbamate group (cleavage conditions: acid) 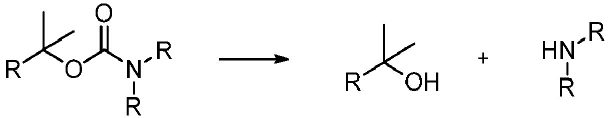
- a acetal or Ketal group (cleavage conditions: acid) 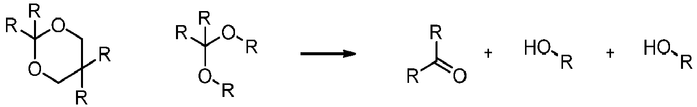
- a siliyl ether group (cleavage conditions: fluoride) 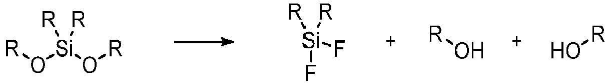
- a N-acetyl sulfonamid group (cleavage conditions: alkylation, nucleophile/hydrolysis) 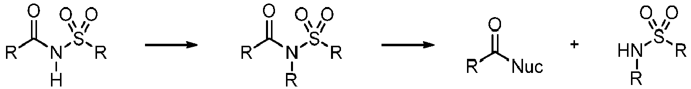
- a acylhydrazone group (cleavage conditions: Acylhydrazine) 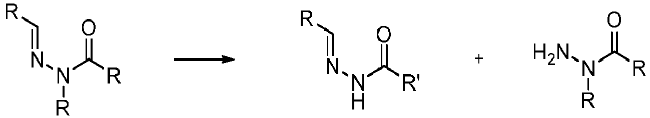
- a levulinoyl ester group (cleavage conditions: hydrazine) 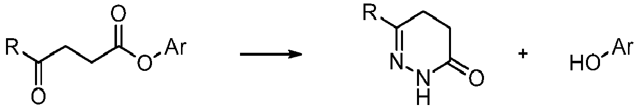
- a phenacyl ester azo group (cleavage conditions: photo) 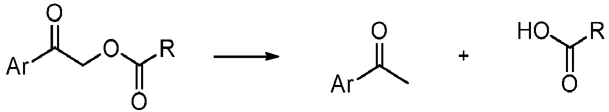
- a tartaric acid groupe (cleavage conditions: oxidative)

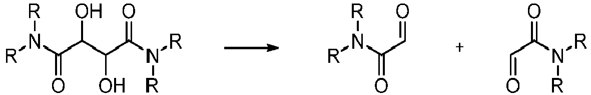

The abbreviations R and Ar on the left side of the reaction scheme represent the further structures of the compounds of the invention (e.g. -S⁰-X or -S-Z-). The compounds on the right side of the reaction scheme are the chemical structures F' resulting from the cleavage reaction. The conditions of the before mentioned cleavage reactions are known to a general expert in the field.

In some embodiments, a cleavable function F, is selected from
- a diarylazo group (Scheme 7, a), which is cleaved by an aqueous sodium dithionite solution (see M. Fonovic, S. H. L. Verhelst, M. T. Sorum, M. Bogyo, Mol. Cell. Proteomics 2007, 6, 1761-1770),
- a disulfide (Scheme 7, b), which is cleaved between the disulfide bridges by thiols, e.g. thioethanol, (see K. Nikolics, E. Szonyi, J. Ramachandran, Biochemistry 1988, 27, 1425-1432), or
- silyloxy groups (Scheme 7, c), which can be opened with fluorides (Fluorides are mild reagent to cleave silyloxy groups in many solvent systems (T. W. Greene, P. G. M. Wuts, Protective Groups in Organic Synthesis, 3rd edition, John Wiley & Sons, New York, 1999).
   a) 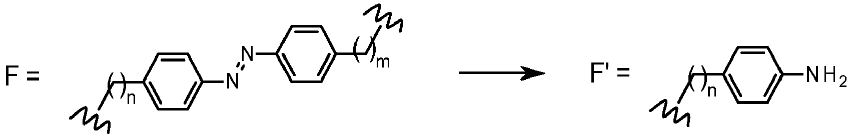
   b) 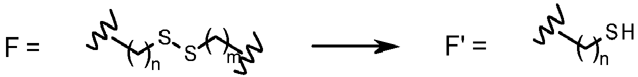
   c)

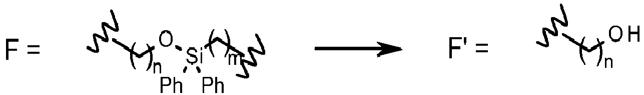

### Scheme 7: Possible cleavable functions F; cleavable under reaction conditions a) buffered Na-dithionite solution;b) thiols, such as thioethanol or DTT or c) fluoride solutions.

The use of a cleavable function allows to selectively remove the captured proteins from the macroscopic carrier B (prior or after a digestion step) and separate the cleaved moiety containing the target compound from the macroscopic carrier B. The advantage is obvious, since proteins, unspecifically bound to the macroscopic carrier B by for example unspecific crosslinking to native proteins would not be removed and therefore isolated together with the specifically captured proteins based on the affinity of Y to the target proteins T. As a result of this cleavage the ratio of specific to unspecific proteins will be improved. Furthermore, the remaining part of the capture compound, to which the proteins are attached, comprises significantly reduced size, in comparison to known cleavable capture compounds after cleavage. Due to position of the cleavable function in proximity to the reactivity function X, not only the macroscopic carrier B but also the selectivity function Y and the remaining necessary linkers S together with the core Z are separated (with the exception of S°). This allows an easier and better characterization of the target compound, due to lesser fragment parts in case of mass spectroscopy. In particular, the determination of the respective crosslink site of the target compound is improved or even - in some cases - possible for the first time with such a cleavable site. The functions, which remain on the cross link site after cleavage (X', F' and/or S°), may a marker, in particular an isotopic marker, a mass tag, fluorescent tag and/or a multiplex marker.

In some embodiments, B is a macroscopic carrier, which is capable of being separated from a medium by means of his physical properties. The macroscopic carrier B allows removing the immobilized compound IC of formula 1 from the biological sample (in particular a mixture of proteins) selectively, for example by virtue of its magnetic, electrostatic, inductive or optical or particulate properties, e.g. by filtration or centrifugation..

In general, the macroscopic carrier B can comprise any solid material. For example, silica gel, glass (e. g., controlled- pore glass), nylon, Wang resin, Merrifield resin, dextran cross-linked with epichlorohydrin (e. g., Sephadex), agarose (e. g., Sepharose), cellulose, magnetic beads, Dynabeads, a metal surface (e. g., steel, gold, silver, aluminum, silicon and copper), a plastic material (e. g., polyethylene, polypropylene, polyamide, polyester, polyvinylidenedifluoride (PVDF)).

The macroscopic carrier B is in any desired form, including, but not limited to, a bead, capillary, plate, membrane, wafer, comb, pin, a wafer with pits, an array of pits or nanoliter wells and other geometries and forms known to those of skill in the art. Thus, the macroscopic carrier B can be particulate or can be in the form of a continuous surface, such as a microtiter plate, a dish or well, a glass slide, a silicon chip, a nitrocellulose sheet, nylon mesh, or other such materials. When particulate, typically the particles have at least one dimension in the 100 µm range or smaller. Such particles, referred collectively herein as "beads", are often, but not necessarily, spherical. Reference to "bead", however, does not constrain the geometry, which can be any shape, including random shapes, needles, fibers, pin (including an array of pins suitable for combinatorial synthesis or analysis) and elongated. "Beads", particularly microspheres that are sufficiently small to be used in the liquid phase, are also contemplated. The "beads" can include additional components, such as magnetic or paramagnetic particles (see, e. g., Dyna beads (Dynal, Oslo, Norway)) for separation using magnets, as long as the additional components do not interfere with the methods and analyses herein.

In some embodiments, B comprises a polymer material or a monolithic polymer material

In some embodiments, B comprises monolithic silica or polymer silica material. In some embodiments, B comprises CPG (Controlled Porous Glass).

In some embodiments, B comprises an in aqueous media non-swellable or low swellable material.

In some embodiment, B comprises magnetic properties.

In some embodiment, B comprises a magnetic particle(s).

In some embodiment, B comprises an anti-fouling material or anti-fouling coating.

In some embodiment, B comprises the form of non-porous beads, porous beads, flat supports, flat supports with pits, arrays, in particular micro arrays, glass, silicon tubes, chips, channels or hollow fibres, pipette tips or microtiter plates.

In some embodiment, B comprises the form of non-porous beads, porous beads, flat supports, flat supports with pits, arrays, in particular micro arrays, glass, silicon tubes, chips, channels or hollow fibres, pipette tips or microtiter plates and a material selected from polymer material, monolithic silica or polymer silica material, monolithic polymer material, CPG (Controlled Porous Glass), in particular an in aqueous media non-swellable or low swellable material. Additionally, B may comprise magnetic properties, in particular magnetic particle(s), and/or an anti-fouling material or anti-fouling coating.

In some embodiment, B comprises "beads" (i. e. particles, typically in the range of less than 200 um or less than 50 pm in their largest dimension) including, but not limited to, polymeric, monolithic silica or polymeric silica material, magnetic, and other such beads. The beads can be made from hydrophobic materials, including polystyrene, polyethylene, polypropylene or teflon, or hydrophilic materials, including, but not limited to, cellulose, dextran cross-linked with epichlorohydrin, agarose, polyacrylamide, silica gel and controlled pore glass beads or particles. These types of capture compounds bound to B can be reacted in liquid phase in suspension, and then spun down or otherwise removed from the reaction medium.

The first linking function D is capable of forming a covalent bond selectively with a second linking function E under reaction conditions not leading to a covalent reaction of D or E with natural occurring polypeptides, in particular with proteins. It has to be noted that the abbreviation spacer moiety S' of the immobilized compound IC of formula 1, formula 1a or 1 a', formula 1 b or 1 b' or formula 6 comprises the spacer moieties S'" of the capture compound CC formula 2, the protein capture compound PCC of formula 5 or 5'or the protein fragment capture compound PFCC of formula 5a or 5a' and spacer moieties S^{iv} of the carrier compound CCB of formula 3 and additionally the structural element D' and E' derived from the reaction of the linking functions D and E. The spacer moiety S' may also be described by the following formula 7 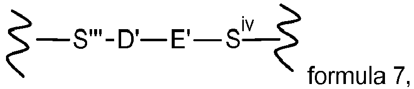 wherein S'" is connected to Z and S'" and S^{iv} have the same meaning as defined previously and D' and E' represent structural elements (fragments) derived from the reaction of the linking functions D and E.

The reaction partners D and E that mediate coupling of the first and second compound of the invention can be any variants of the reactions commonly referred to as "click" chemistry.

The reaction most often used to date in biological chemistry is the 1,3-dipolar cycloaddition reaction (scheme 8, a) between an azide and an alkyne developed by Huisgen (Rostovtsev et al., Angew. Chem. 2002; van Berkel et al., ChemBioChem 2008, 9, 1805-1815. Another possibility is a Staudinger ligation (Saxon & Bertozzi, Science 2000, 287, 2007-2010; Köhn & Breinbauer, Angew. Chem. 2004, 116, 3168-3178; Scheme 1 b upper panel) between an azide and an arylcarboxylic acid methyl ester-5-ortho-phosphine or a Staudinger phosphite reaction (Serwa et al., Angew. Chem. 2009, 121 Scheme 1 b lower panel) between an azide and a phosphite ester (Scheme 8, b).

Alternatively a Diels-Alder reaction (Scheme 8, c) with the ene component as the linking function D (Song et al., Angew. Chem. 2008, 120, 2874-2877; J. Am. Chem. Soc. 2008, 130, 9654-9655; Devaraj et al., Bioconjugate Chem. 2008, 19, 2297-2299 or N. K. Devaraj, R. Weissleder, S. A. Hilderbrand, Bioconjugate Chem. 2008, 19, 2297-2299) may be applied.

A further alternative (Scheme 8, d) may be the use of a Diels-Alder reaction with the ene component as the linking function E (no catalyst: V. Marchán, S. Ortega, D. Pulido, E. Pedroso, Anna Grandas, Nucl. Acids Res. 2006, 34, e24 1-9; Titanium catalyst: K. E. Litz, Molecules 2007, 12, 1674-1678 or A. Serganov, S. Keiper, L. Malinina, V. Tereshko, E. Skripkin, C. Höbartner, A. Polonskaia, A. T. Phan, R. Wombacher, R. Micura, Z. Dauter, A. Jäschke, D. J. Patel, Nat. Struct. Mol. Bio/. 2005, 12, 218-224).

Another alternative (see Scheme 8, e) may be the use of an ene reaction (K. L. Killops, L. M. Campos, C. J. Hawker, J. Am. Chem. Soc. 2008, 130, 5062-5064) or photo-thio-en reaction (S. Narayan, J. Muldoon, M. G. Finn, V. V. Fokin, H. C. Kolb, K. B. Sharpless, Angew. Chem. 2005, 117, 3339-3343), which can be carried out in the caproBox^{™} due to a photo-induced click process at 300-400 nm.
a) 1,3-dipolar cycloaddition 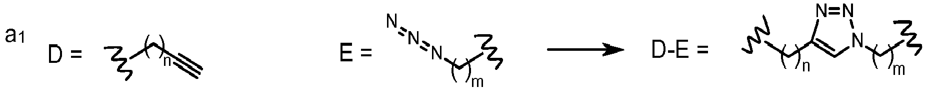 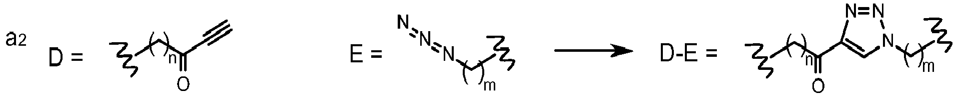
b) Staudinger ligation and Staudinger phosphite reactions 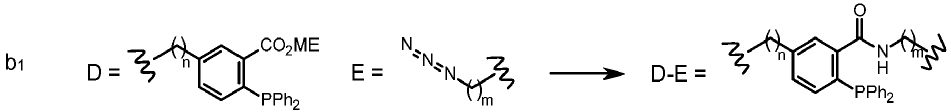 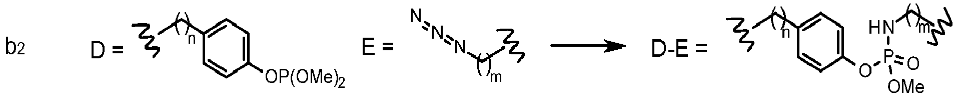
c) Diels-Alder reactions (D = alkene) 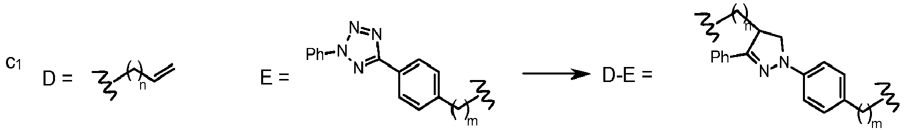 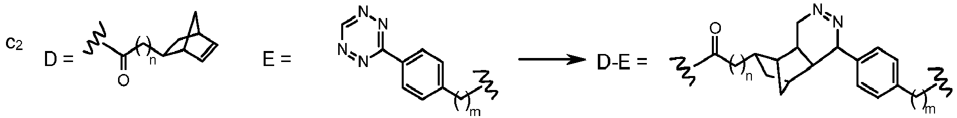
d) Diels-Alder reactions (D = diene) 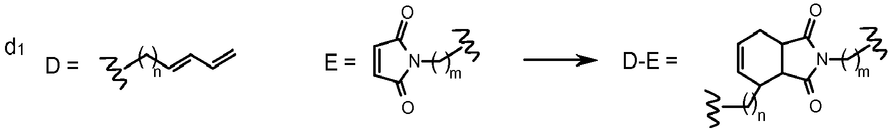 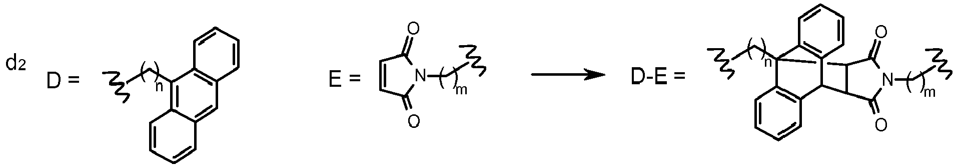
e) Examples for further coupling reactions (ene reactions)

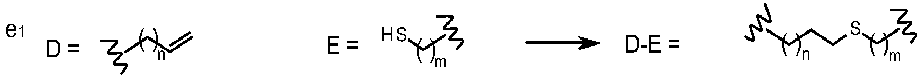

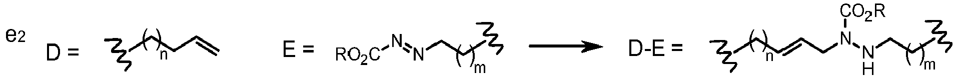

Scheme 8: Possible linking groups D and E; reaction conditions concerning a linking reaction of said linking groups D and E; a₁: CuSO₄, sodium ascorbate; a₂: no catalyst/additive; b₁: no catalyst/additive; b₂: no catalyst/additive; c₁: h·v (λ = 302 nm); c₂: no catalyst/additive; d₁: no catalyst/additive or metal salt catalysis; d₂: Diels-Alderase (ribozyme); e₁: h·v (λ = 350 nm); e₂: no catalyst/additive.

According to some embodiments, D and E are each selected together from the group of reaction partners, which are capable of performing a reaction according to the click chemistry, wherein in particular
a. the reaction partners comprise
   - an azide for one reaction partner and
   - a member of the group comprising alkyne, aryl carboxylic acid methylester- ortho-phosphine, phosphite ester or a chelatising ligand for the other reaction partner or
b. the reaction partners comprise
   - a triple bond or a double bond, particularly a cyclic double bond, such as for example norbornene or cyclooctene or a derivative thereof for one reaction partner, and
   - a tetrazine or tetrazine derivative for the other reaction partner.

In other words, either D or E is an azide, and the other partner is a member of the group comprising alkyne, aryl carboxylic acid methylester- ortho-phosphine, phosphite ester or a chelatising ligand. Alternatively, either D or E is a tetrazine or tetrazine derivative and the other partner comprises a triple bond or a double bond, particularly a cyclic double bond, such as for example norbornene or cyclooctene or a derivative thereof.

In some embodiments, either D or E is selected from the following tetrazines 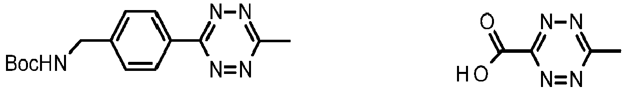 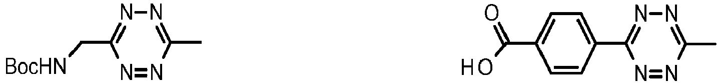 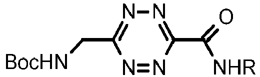 and the other partner comprises a triple bond or a double bond.

The reactivity function X may be connected to the cleavable function F by a spacer moiety S°. The selectivity function Y, the cleavable function F and the linking function D may be connected to the central core Z by a spacer moiety S" (for Y), S (for F) and S'" (for D). The linking function E may be connected to the macroscopic carrier B by a spacer moiety S^{iv}. Furthermore, the macroscopic carrier B is connected to the central core Z by the spacer moiety S', wherein the spacer moiety S' comprises at least the structural elements D' and E' - derived from the reaction of the linking functions D and E - and can comprise the optional spacer moieties S'" and S^{iv}. Thus, S' is predefined by the linking functions D and E and the optional spacer moieties S'" and S^{iv}.

The spacer moieties S⁰, S", S'" and S^{iv} can be any group that provides a spacing (a distance between two functions). Thus, the spacer S⁰, S", S'" and S^{iv} (and in case of formula 1 the spacer S') can be any chemical moiety that is suited to link the functional moieties X, F, Y and D in formula 2, E and B in formula 3 and X, F, Y and B, for formula 1 with each other or the central core Z.

In some embodiments, each S⁰, S", S'" and S^{iv} are an aliphatic chain or a heteroalkylchain, which comprises carbon and nitrogen, oxygen, sulphur, silicon and/or phosphorous atoms, wherein in particular said heteroalkyl chain comprises at least one oxygen atom, optionally the aliphatic chain or the heteroalkyl chain comprise one or more carbon double or triple bonds.

In some embodiments, at least one S⁰, S", S'" and S^{iv} an aliphatic chain or a heteroalkylchain, which comprises carbon and nitrogen, oxygen, sulphur, silicon and/or phosphorous atoms, wherein in particular said heteroalkyl chain comprises at least one oxygen atom, optionally the aliphatic chain or the heteroalkylchain comprise one or more carbon double or triple bonds.

In some embodiments, each S⁰, S", S'" and S^{iv} comprises chain length of 10 to 20 carbon atoms, in particular of 3 to 10 carbon atoms. The chain may comprise substituents and/or any carbon atom of the chain may be substituted by hetero atoms and contains the hydrogen atoms corresponding to the oxidation state of each non-hydrogen atom. One example for a spacer moiety is the methylenoxy moiety forming the side chain of serine, a CO-NH or NH-CO moiety (linkage by amide on the nitrogen or carboxy carbon), a CO-O or O-CO moiety. One particular example is a PEG-spacer moiety (polyethylene glycol polymer or oligomer). When designing compounds of formula 1, 2 or 3, ease of chemical synthesis, sterical freedom of functions X, F, Y, D, E and B, particularly the interaction of Y with the protein to be isolated, and the requirements regarding diffusion into cells or other biological structures will be taken into account.

Those of skill in the art in the light of the disclosure herein can readily select suitable spacers.

In some embodiments, the spacer moieties S⁰, S", S'" and S'" is selected from (CH₂)ᵣ, (CH₂O), (CH₂CH₂O)ᵣ, (NH(CH₂)ᵣC(=O))ₛ, (O(CH)ᵣC(=O))ₛ,- ((CH₂)ᵣ₁-C(O)NH-(CH2)ᵣ₂)ₛ- and-(C(O)NH-(CH₂)ᵣ)_{S} , where r, r1, r2 and s are each independently and integer from 1 to 10.

Examples for spacer moieties S⁰, S", S'" and S^{iv} are 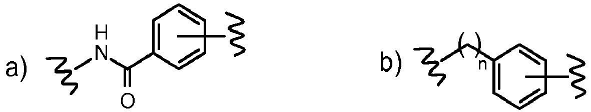 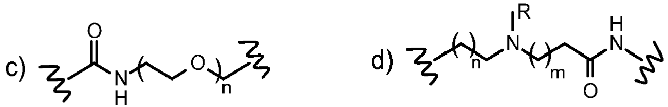 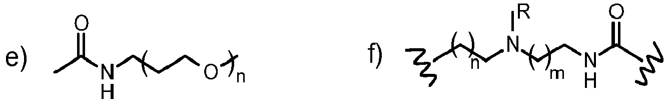 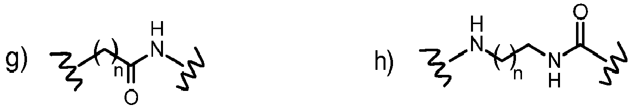 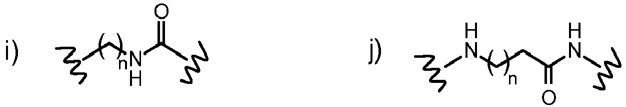 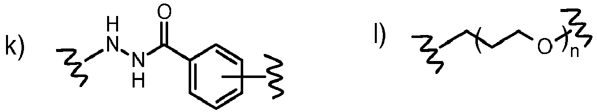 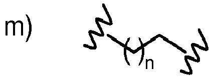 with n = 0 to n = 20.

X and Y should be linked to Z (via F in case of X) by spacer moieties that are sufficiently long to enable both X and Y functions to interact with protein surfaces independently from each other; on the other hand spacer moieties should be minimized to allow for diffusion into cells or organelles, where such diffusion is expected.

In general, Z is the central atom or core bridging the moieties F (and thus X), Y and D in the capture compound CC of formula 2 (and thereby, B in the immobilized compound IC of formula 1 after reaction with the carrier compound CCB of formula 3).

In some embodiments, Z is carbon, nitrogen, phosphorus, silicon, sulfonium, cycloalkyl, unsaturated cycloalkyl, unsaturated hetero cycloalkyl or heteroaryl.

The functional moieties X, F, Y, D/E, and B are linked to each other by bridging moieties (spacers), as discussed previously. In its most simple manifestation, Z is a carbon atom, for example the central carbon atom of an alpha amino acid. Proteinogenic amino acids having a side chain such as lysine, glutamic or aspartic acid or cysteine or serine are suitable core molecules since they provide three functions that can be easily derivatized and coupled to moieties independently from each other. If, for instance, lysine is used, then the central alpha carbon atom can be viewed as the core Z, and the carboxy function, the amino function and the butylamine function can be viewed as the respective spacer moieties. If, for instance, glutamic acid is used, then the central alpha carbon atom can be viewed as the core Z, and the carboxy function, the amino function and the propionic acid function can be viewed as the respective spacer moieties.

Similarly, Z can be a tertiary amine bearing the three functions F (and thus X), Y and D.

In some embodiments, Z is a trisubstituted phosphorus atom, for example a phosphine

In some embodiments, Z is a silicium atom.

In some embodiments, the core Z comprises a ring structure such as a cycloalkyl, unsaturated cycloalkyl, unsaturated heterocycloalkyl or heteroaryl.

The reactivity function X can be attached directly to the cleavable function F or can be attached via a spacer moiety S° to the cleavable function F.

In some embodiments, S° is selected from a polyethylene glycol polymer or oligomer.

In some embodiments, S° comprises a chain length of up to 20 carbon atoms optionally including heteroatoms such as O, N, S, Si or P and one or more double or triple bonds.

In some embodiments, S° comprises a chain length of up to 10 carbon atoms optionally including heteroatoms such as O, N, S, Si or P and one or more double or triple bonds.

In some embodiments, S° comprises a chain length of up to 6 carbon atoms optionally including heteroatoms such as O, N, S, Si or P and one or more double or triple bonds.

In some embodiments, the reactivity function X is attached directly to the cleavable function F in order to minimize the size of the moiety which remains attached after cleavage of F at the captured protein or peptide fragment to facilitate the subsequent analysis by mass spectrometry.

The selectivity function Y can be attached directly to the central core Z or can be attached via a spacer moiety S" to the central core Z.

In some embodiments, S" is a polyethylene glycol polymer or oligomer.

In some embodiments, S" comprises a chain length of up to 20 carbon atoms optionally including heteroatoms such as O, N, S or P and one or more double or triple bonds In some embodiments, S" comprises a chain length of up to 10 carbon atoms optionally including heteroatoms such as O, N, S or P and one or more double or triple bonds.

In some embodiments, S" comprises a chain length of up to 6 carbon atoms optionally including heteroatoms such as O, N, S or P and one or more double or triple bonds.

In some embodiments, the selectivity function Y is attached directly to the central core Z.

In some embodiments, the spacer moiety S" is chosen such that the selectivity function can reach the binding pocket of a target or non-target protein.

The cleavable function F can be attached directly to the central core Z or can be attached via a spacer moiety S to the central core Z.

In some embodiments, S is a polyethylene glycol polymer or oligomer.

In some embodiments, S comprises a chain length of up to 20 carbon atoms optionally including heteroatoms such as O, N, S, Si or P and one or more double or triple bonds In some embodiments, S comprises a chain length of up to 10 carbon atoms optionally including heteroatoms such as O, N, S, Si or P and one or more double or triple bonds.

In some embodiments, S comprises a chain length of up to 6 carbon atoms optionally including heteroatoms such as O, N, S, Si or P and one or more double or triple bonds.

In some embodiments, the cleavable function F is attached directly to the central core Z.

The linking function D can be attached directly to the central core Z or can be attached via a spacer moiety S to the central core Z.

In some embodiments, S'" is a polyethylene glycol polymer or oligomer.

In some embodiments, S"' comprises a chain length of up to 20 carbon atoms optionally including heteroatoms such as O, N, S, Si or P and one or more double or triple bonds.

In some embodiments, S'" comprises a chain length of up to 10 carbon atoms optionally including heteroatoms such as O, N, S, Si or P and one or more double or triple bonds.

In some embodiments, the linking function D is attached directly to the central core Z.

The linking function E can be attached directly to macroscopic carrier B or can be attached via a spacer moiety S^{iv} to macroscopic carrier B.

In some embodiments, S^{iv} is a polyethylene glycol polymer or oligomer.

In some embodiments, S^{iv} comprises a chain length of up to 20 carbon atoms optionally including heteroatoms such as O, N, S, Si or P and one or more double or triple bonds.

In some embodiments, S^{iv} comprises a chain length of up to 10 carbon atoms optionally including heteroatoms such as O, N, S, Si or P and one or more double or triple bonds.

In some embodiments, the cleavable function F is attached directly to the central core Z.

In some embodiments, S' (derived from the reaction of -S"'-D and E-S^{iv}-; see formula 7 and discussion above) comprises a chain length up to 40 atoms, wherein the nature of the chain is depending on the functions S"', D', E' and S^{iv} (as discussed previously)..

In some embodiments, the functions X, X', F, F' and/or S° comprise a marker, in particular an isotopic marker, a mass tag, fluorescent tag and/or a multiplex marker. It has to be noted, that a marker can be produced by a cleavage of the cleavable function F yielding the function F', which can now act after the cleavage as a marker.

Any combination of the structural elements X, X', Z, P, PF, BM, BMF, S°, S, S",S"', S'", Y, B, D, E and F - independently from each other - discussed in the previous section is possible for providing the compounds of the invention or for the process of the invention. A general expert in the field may choose suitable combinations. Reference is also made to the examples in the following sections. The structural elements or similar elements thereof (e.g. F, X, Y ...) of the compounds depicted or mentioned below may also be used in a combination - independently from each other - for providing compounds of the invention or for the process of the invention.

In some embodiments, the characterization step is achieved by applying mass spectrometry, in particular matrix assisted laser desorption ionization (MALDI), continuous or pulsed electrospray ionization (ESI), ionspray, thermospray, or massive cluster impact mass spectrometry for ionization, and linear time-of-flight (TOF), reflectron time-of-flight, single quadrupole, multiple quadrupole, single magnetic sector, multiple magnetic sector, Fourier transform ion cyclotron resonance (ICR), orbitrap or ion trap for mass analysis.

According to a second aspect of the invention provided herein is a carrier compound CCB, particularly a carrier compound CCB for carrying out a process according to the first aspect of the invention, of the general formula 3, 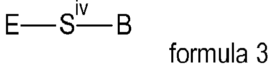 wherein S^{iv} comprises a polyethylene glycol group,
- B is a macroscopic carrier, in particular B comprises CPG, monolithic silica, polymer silica or a polymer material or a monolithic polymer material, wherein in particular B comprises additionally magnetic properties, and
- E comprises a carbon-carbon double bond or a carbon-carbon triple bond, in particular a cyclic double bond, such as for example norbornene or cyclooctene, tetrazine or a derivative of the aforementioned compounds.

Concerning specific embodiments of the functions E and B and the spacer moiety S^{iv} reference is made to the previously discussed embodiments with respect to the first aspect of the invention.

Examples of a carrier compound CCB are:

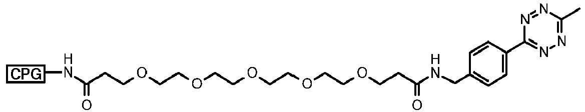

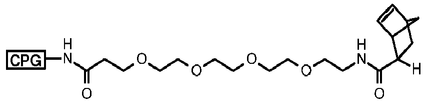

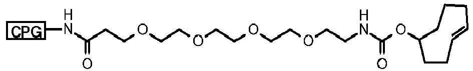

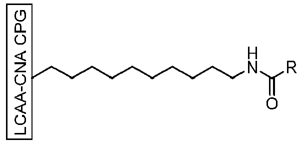

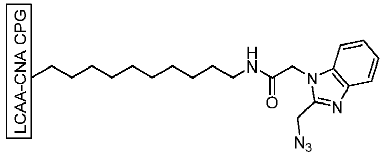

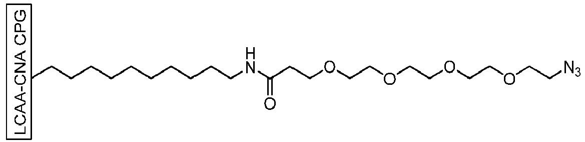

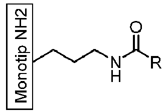

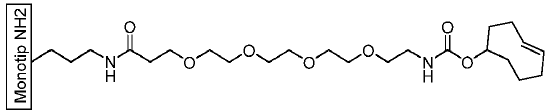

LCAA refers to a long chain aminoalkyl, CNA refers to long chain alkyl amine and CPG refers to Controlled Porous Glass. Reference is made to the experimental section. R refers to a second linking function E, as discussed previously and as depicted in the examples.

According to a third aspect of the invention provided herein is a capture compound CC, particularly a capture compound CC for carrying out a process according to the first aspect of the invention, of the general formula 2, 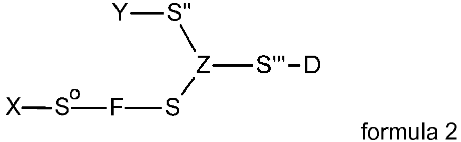 wherein
- X is a reactivity function which can be activated by irradiation and can form after said activation a covalent bond to a target compound T,
- F is a cleavable function,
- Y is a selectivity function selected from the group comprising a bioactive small molecule,
- Z is carbon, nitrogen, phosphorus, silicon, sulfonium, cycloalkyl, unsaturated cycloalkyl, unsaturated heterocycloalkyl or heteroaryl,
- D is a first linking function capable of forming a covalent bond selectively with a second linking function E under reaction conditions not leading to a covalent reaction of D or E with natural occurring polypeptides, in particular with proteins,
- S°, S, S" and S'" are spacer moieties, each of which represents, independently of the others, a bridge between X and F, Z and F, Z and Y or Z and D, facilitated by covalent bonds of carbon, nitrogen, oxygen, sulphur, silicon and/or phosphorous atoms, wherein S°, S, S" and S'" are optional.

Concerning specific embodiments of the functions X, F, Y, Z and D and the spacer moieties S°, S, S" and S'" reference is made to the previously discussed embodiments with respect to the first aspect of the invention.

Examples of a capture compound CC are:
- capture compounds CC comprising a N-acetyl sulfonamide group 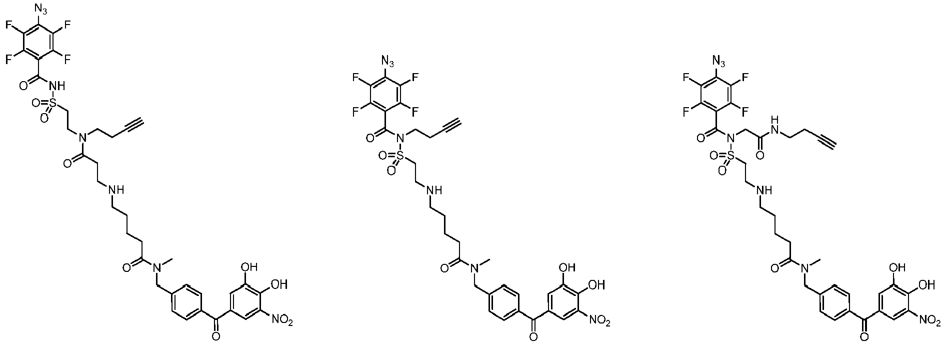
- capture compounds CC comprising a carbamate group 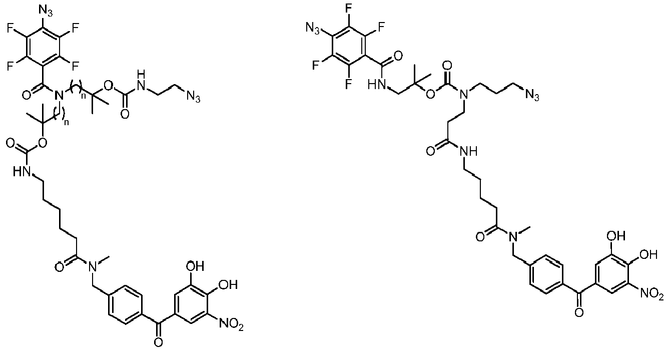
- capture compounds CC comprising a acetale group 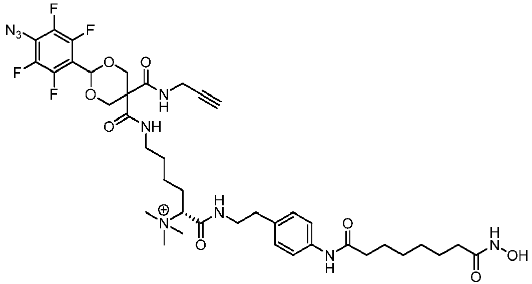
- capture compounds CC comprising an acylhydrazone group 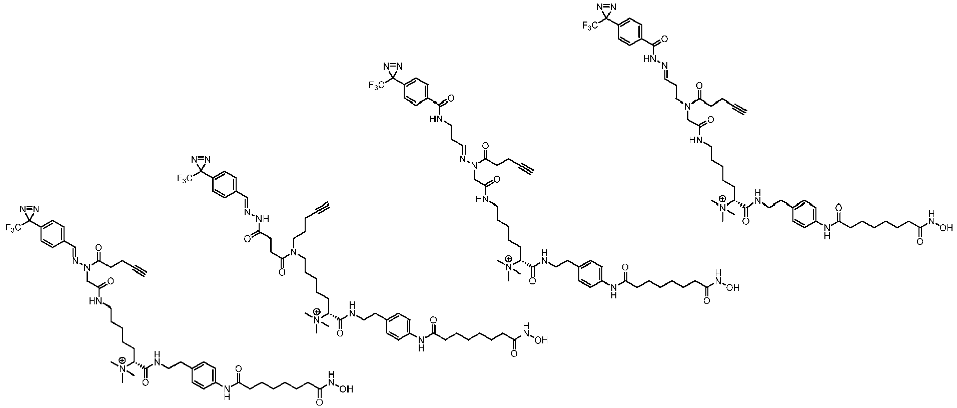
- capture compounds CC comprising an azo group

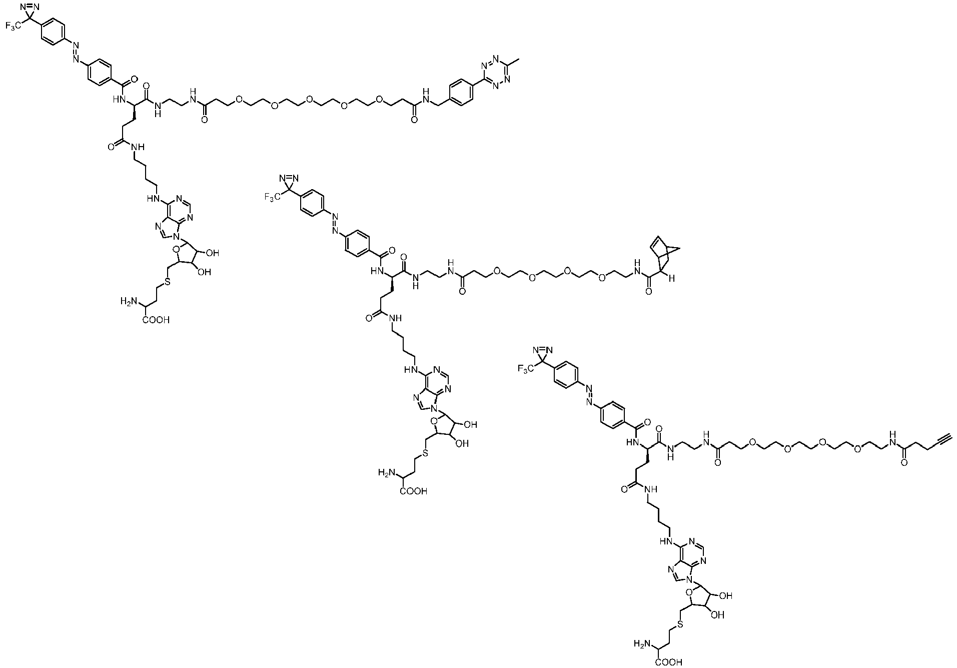

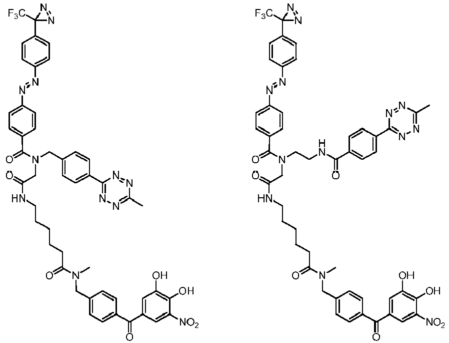

Reference is made to the experimental section.

According to a fourth aspect of the invention provided herein is a precursor compound PC, particularly a precursor compound PC for carrying out a process according to the first aspect of the invention, of the general formula 6, 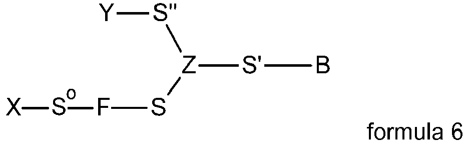 wherein
- X is a reactivity function which can be activated by irradiation and can form after said activation a covalent bond to a target compound T,
- F is a cleavable function,
- Y is a selectivity function selected from the group comprising a bioactive small molecule, in particular
- Y is a bioactive small molecule selected from the group comprising a pharmaceutical drug, a drug development candidate, a drug fragment, a drug metabolite, a prodrug, a herbicide, a fungicide, an insecticide or a natural product, wherein in particular all the before mentioned selectivity functions Y are low-molecular weight compounds, more particularly a pharmaceutical drug, a drug fragment, a drug metabolite, a prodrug, a herbicide or a natural product with less than 1500 u, in particular less than 1000 u, more particularly less than 500 u, in molecular mass,
- Z is carbon, nitrogen, phosphorus, silicon, sulfonium, cycloalkyl, unsaturated cycloalkyl, unsaturated heterocycloalkyl or heteroaryl,
- B is a macroscopic carrier, which is capable of being separated from a medium by means of his physical properties,
- S°, S, S' and S" are spacer moieties, each of which represents, independently of the others, a bridge between X and F, Z and F, Z and B or Z and Y, facilitated by covalent bonds of carbon, nitrogen, oxygen, sulphur, silicon and/or phosphorous atoms, wherein S°, S and S" are optional.

Concerning specific embodiments of the functions X, F, Y, Z and B and the spacer moieties S°, S, S' and S" reference is made to the previously discussed embodiments with respect to the first aspect of the invention.

According to a fifth aspect of the invention provided herein is a division compound DC, particularly a division compound DC for carrying out a process according to the first aspect of the invention, of the general formula 4, 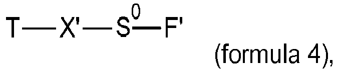 in particular a division compound DCb of the general formula 4b or 4b' 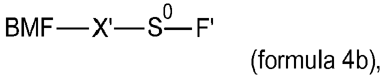 more particularly 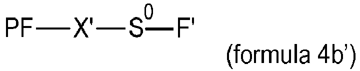 wherein
- X' is the chemical structure resulting from activation of the reactivity function X by irradiation and the subsequent forming of a covalent bond to the target compound T
- T is a target compound comprising a biomolecule BM, in particular protein P, or a biomolecule fragment BMF, in particular a protein fragment PF, with BM, P, BMF and PF having the meanings defined previously,
- S° is a spacer moiety, which represents a bridge between X' and F', facilitated by covalent bonds of carbon, nitrogen, oxygen, sulphur, silicon and/or phosphorous atoms, wherein S° is optional,
- F' is the chemical structure resulting from a cleavage reaction of a cleavable function F, wherein the cleavable function F is cleavable by H⁺, OH⁻, thiols, salts from acids, fluorides, hydrazides, nucleophiles, electrophiles, radicals, alkylating agents, oxidation, reduction, enzymes or irradiation or a combination of the before mentioned, in particular F is selected from the group comprising an azo-, an acyl-, a disulfide-, a silyoxy-, a carbamate- , an acetal-, a ketal, an arylether, a hydrazone-, an arylketone or a o-nitrobenzyl group.

Concerning specific embodiments of the functions X', P, PF and F' and the spacer moiety S° reference is made to the previously discussed embodiments with respect to the first aspect of the invention.

According to a sixth aspect of the invention provided herein is a combination of reagents for carrying out a process according to the first aspect of the invention, in particular a combination of reagents for carrying out a process according to at least one of the claims 1 to 12, comprising a first compound according to claim 13 and a second compound according to claim 14.

Concerning specific embodiments of the combination of reagents and the use of the combination of reagents for a process according to the invention reference is made to the previously discussed embodiments with respect to the first, second and third aspect of the invention.

Detailed examples, results and conditions are provided in the example section. The methods, steps and conditions concerning the capturing of biomolecules, in particular proteins is known in the art and can be considered basic knowledge of the general expert in the field. Reference is made to the detailed description of the EP 1 485 707 B1, in particular page 43 to 61 and examples 1 to 9.

### Experimental section:

### Synthesis of carrier compound CCB:

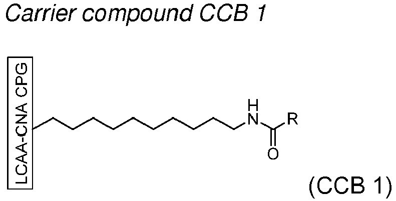

**Synthesis.** In a Pierce® Spin Column a solution of R-COOH (2.2 eq.), DIPEA (5 eq.) and HATU (2.0 eq.) in dry DMA (300 µL) was rotated in a VWR tube rotator for 10 min at 23 °C. LCAA/CNA CPG (1.0 eq., Prime Synthesis, 57.0 µmol(NH₂)/g, 3000 A, 120-200 mesh) were added and rotation were continued for 1 h at 23 °C. The reaction solution was flushed out and the LCAA/CNA CPG was successively washed with DMA, MeOH and DCM. The LCAA/CNA CPG was incubated with a solution of 1.0 mL acetic anhydride and pyridine (4/1) and rotated for 1 h at 23 °C. The reaction solution was flushed out and the LCAA/CNA CPG was successively washed with H₂O, DMA, MeOH and DCM.

**Loading determination.** A sample of the LCAA/CNA CPG was incubated with a solution of 2-(6-(dimethylamino)-3-(dimethyliminio)-3H-xanthen-9-yl)-5-(prop-2-yn-1-ylcarbamoyl)benzoate [Probe 1] (1.0 eq.), Copper(II) sulfate (1.0 eq.) and Sodium ascorbate (2.5 eq.) in a solvent mixture of H₂O and MeOH (150 µL, 1/1) and rotated for 1 h at 23 °C. The reaction solution was flushed out and the LCAA/CNA CPG was successively washed with H₂O and MeOH. The reaction solution and the washing solutions were combined and diluted with water. Loading of the LCAA/CNA CPG was determined by comparison of the UV absorption at 562 nm (anthos Reader 2010, Typ: 17550) of the solution to a reference.

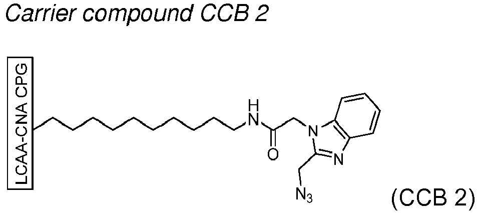

**Synthesis.** In a Pierce® Spin Column a solution of 2-(2-(azidomethyl)-1H-benzo[d]imidazol-1-yl)acetic acid (2.90 mg, 12.5 µmol), DIPEA (5.0 µL, 28.5 µmol) and HATU (4.3 mg, 11.4 µmol) in dry DMA (300 µL) was rotated in a VWR tube rotator for 10 min at 23 °C. 100 mg (5.7 µmol) LCAA/CNA CPG (Prime Synthesis, 57.0 µmol(NH₂)/g, 3000 A, 120-200 mesh) were added and rotation were continued for 1 h at 23 °C. The reaction solution was flushed out and the LCAA/CNA CPG was successively washed with DMA, MeOH and DCM. The LCAA/CNA CPG was incubated with a solution of 1.0 mL acetic anhydride and pyridine (4/1) and rotated for 1 h at 23 °C. The reaction solution was flushed out and the LCAA/CNA CPG was successively washed with H₂O, DMA, MeOH and DCM.

**Loading determination.** A sample of the LCAA/CNA CPG (3.0 mg) was incubated with a solution of 2-(6-(dimethylamino)-3-(dimethyliminio)-3H-xanthen-9-yl)-5-(prop-2-yn-1-ylcarbamoyl)benzoate [Probe 1] (0.08 mg, 0.17 µmol), Copper(II) sulfate (0.04 mg, 0.17 µmol) and Sodium ascorbate (0.09 mg, 0.43 µmol) in a solvent mixture of H₂O and MeOH (150 µL, 1/1) and rotated for 1 h at 23 °C. The reaction solution was flushed out and the LCAA/CNA CPG was successively washed with H₂O and MeOH. The reaction solution and the washing solutions were combined and diluted with water. Loading of the LCAA/CNA CPG was determined to 33.0 µmol(N₃)/g by comparison of the UV absorption at 562 nm (anthos Reader 2010, Typ: 17550) of the solution to a reference.

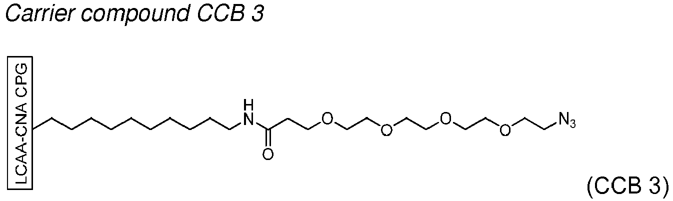

**Synthesis.** In a Pierce® Spin Column a solution of 1-azido-3,6,9,12-tetraoxapentadecan-15-oic acid (166.0 µL, 0.06 µmol), DIPEA (0.05 µL, 0.29 µmol) and HATU (0.02 mg, 0.06 µmol) in dry DMA (300 µL) was rotated in a VWR tube rotator for 10 min at 23 °C. 10 mg (0.57 µmol) LCAA/CNA CPG (Prime Synthesis, 57.0 µmol(NH₂)/g, 3000 A, 120-200 mesh) were added and rotation were continued for 1 h at 23 °C. The reaction solution was flushed out and the LCAA/CNA CPG was successively washed with DMA, MeOH and DCM. The LCAA/CNA CPG was incubated with a solution of 1.0 mL acetic anhydride and pyridine (4/1) and rotated for 1 h at 23 °C. The reaction solution was flushed out and the LCAA/CNA CPG was successively washed with H₂O, DMA, MeOH and DCM.

**Loading determination.** The LCAA/CNA CPG was incubated with a solution of 2-(6-(dimethylamino)-3-(dimethyliminio)-3H-xanthen-9-yl)-5-(prop-2-yn-1-ylcarbamoyl)benzoate [Probe 1] (0.03 mg, 0.06 µmol), Copper(II) sulfate (0.01 mg, 0.05 µmol) and Sodium ascorbate (0.03 mg, 0.13 µmol) in a solvent mixture of H₂O and MeOH (150 µL, 1/1) and rotated for 1 h at 23 °C. The reaction solution was flushed out and the LCAA/CNA CPG was successively washed with H₂O and MeOH. The reaction solution and the washing solutions were combined and diluted with water. Loading of the LCAA/CNA CPG was determined to 2.9 µmol(N₃)/g by comparison of the UV absorption at 562 nm (anthos Reader 2010, Typ: 17550) of the solution to a reference.

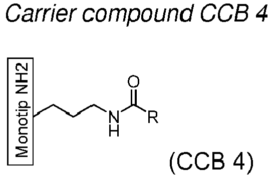

**Synthesis.** A Monotip NH2 (GLScience, 2.0 µmol(NH₂)/tip) was incubated with a solution of (*E*)-2,5-dioxopyrrolidin-1-yl 1-(cyclooct-4-en-1-yloxy)-1-oxo-5,8,11,14-tetraoxa-2-azaheptadecan-17-oate (1.5 eq.) and DIPEA (10 eq.) in dry DMA (150 µL) applying Thermo Finnpipette (200 µL, Up 1, Down 1, Cycles 999). The reaction solution was flushed out. The Monotip NH2 was incubated a second cycle with a solution of (*E*)-2,5-dioxopyrrolidin-1-yl 1-(cyclooct-4-en-1-yloxy)-1-oxo-5,8,11,14-tetraoxa-2-azaheptadecan-17-oate (0.75 eq.) and DIPEA (5.0 eq.) in dry DMA (150 µL) applying Thermo Finnpipette (200 µL, Up 1, Down 1, Cycles 999). The reaction solution was flushed out and the Monotip NH2 was washed with DMA (Thermo Finnpipette - 200 µL, Up 1, Down 1, Cycles 100) and DCM (Thermo Finnpipette - 200 µL, Up 1, Down 1, Cycles 100). The Monotip NH2 was incubated with a solution of 2.5 mL acetic anhydride and pyridine (4/1) applying Thermo Finnpipette (200 µL, Up 1, Down 1, Cycles 999). The reaction solution was flushed out. The Monotip NH2 was washed successively with H₂O, MeOH and DCM (Thermo Finnpipette - 200 µL, Up 1, Down 1, Cycles 100).

**Loading determination.** The Monotip NH2 was incubated with a solution of 3',6'-bis(dimethylamino)-N-(1-(4-(6-methyl-1,2,4,5-tetrazin-3-yl)phenyl)-1-oxo-6,9,12-trioxa-2-azapentadecan-15-yl)-3-oxo-3H-spiro[isobenzofuran-1,9'-xanthene]-5-carboxamide [Probe 2] (0.25 eq.) in a solvent mixture of H₂O and MeOH (150 µL, 1/1) applying Thermo Finnpipette (200 µL, Up 1, Down 1, Cycles 999). A sample of 5 µL was taken out and diluted with 200 µL water. Loading of the Monotip NH2 was determined by comparison of the UV absorption at 562 nm (anthos Reader 2010, Typ: 17550) of the sample to a reference.

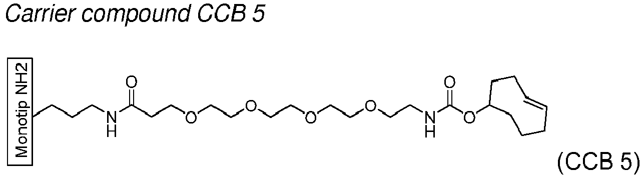

**Synthesis.** A Monotip NH2 (GLScience, 2.0 µmol(NH₂)/tip) was incubated with a solution of (*E*)-2,5-dioxopyrrolidin-1-yl 1-(cyclooct-4-en-1-yloxy)-1-oxo-5,8,11,14-tetraoxa-2-azaheptadecan-17-oate (1.54 mg, 3.0 µmol) and DIPEA (3.49 µL, 20.0 µmol) in dry DMA (150 µL) applying Thermo Finnpipette (200 µL, Up 1, Down 1, Cycles 999). The reaction solution was flushed out. The Monotip NH2 was incubated a second cycle with a solution of (*E*)-2,5-dioxopyrrolidin-1-yl 1-(cyclooct-4-en-1-yloxy)-1-oxo-5,8,11,14-tetraoxa-2-azaheptadecan-17-oate (0.77 mg, 1.5 µmol) and DIPEA (1.75 µL, 10.0 µmol) in dry DMA (150 µL) applying Thermo Finnpipette (200 µL, Up 1, Down 1, Cycles 999). The reaction solution was flushed out and the Monotip NH2 was washed with DMA (Thermo Finnpipette - 200 µL, Up 1, Down 1, Cycles 100) and DCM (Thermo Finnpipette - 200 µL, Up 1, Down 1, Cycles 100). The Monotip NH2 was incubated with a solution of 2.5 mL acetic anhydride and pyridine (4/1) applying Thermo Finnpipette (200 µL, Up 1, Down 1, Cycles 999). The reaction solution was flushed out. The Monotip NH2 was washed successively with H₂O, MeOH and DCM (Thermo Finnpipette - 200 µL, Up 1, Down 1, Cycles 100).

**Loading determination.** The Monotip NH2 was incubated with a solution of 3',6'-bis(dimethylamino)-N-(1-(4-(6-methyl-1,2,4,5-tetrazin-3-yl)phenyl)-1-oxo-6,9,12-trioxa-2-azapentadecan-15-yl)-3-oxo-3H-spiro[isobenzofuran-1,9'-xanthene]-5-carboxamide [Probe 2] (0.49 mg, 0.5 µmol) in a solvent mixture of H₂O and MeOH (150 µL, 1/1) applying Thermo Finnpipette (200 µL, Up 1, Down 1, Cycles 999). A sample of 5 µL was taken out and diluted with 200 µL water. Loading of the Monotip NH2 was determined to 0.5 µmol(TCO)/tip by comparison of the UV absorption at 562 nm (anthos Reader 2010, Typ: 17550) of the sample to a reference.

### Synthesis of Probe 1.

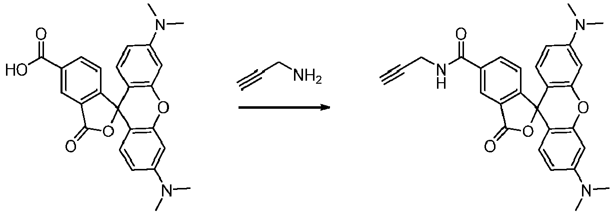

A solution of 3',6'-bis(dimethylamino)-3-oxo-spiro[isobenzofuran-1,9'-xanthene]-5-carboxylic acid [TAMRA] (40.0 mg, 0,1 mmol) and Diisopropylethylamin [DIPEA] (81.0 µl, 0,47 mmol) in dry DMA (1.0 mL) was treated with prop-2-yn-1-amine (6.6 µL, 0.1 mmol) and 1-[Bis(dimethylamino)methylene]-1H-1,2,3-triazolo[4,5-b]pyridinium 3-oxid hexafluorophosphate [HATU] (35.3 mg, 0.1 mmol). The reaction mixture was stirred for 30 min at 23 °C. The solvent was removed under reduced pressure and the crude product was purified by column chromatography to yield probe 1 (25.4 mg, 0.05 mmol, 59%) as a red solid.

### Synthesis Probe 2.

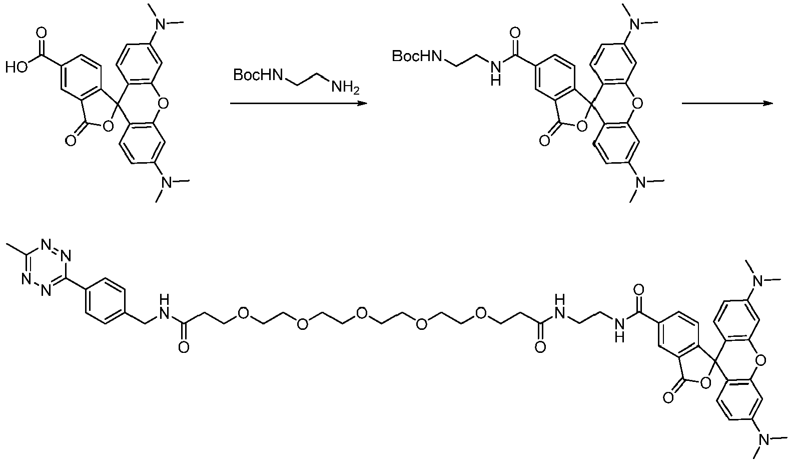

A solution of TAMRA (40.0 mg, 0.1 mmol) and tert-butyl (2-aminoethyl)carbamate (16.4 mg, 0.1 mmol) was treated with DIPEA (81.0 µl, 0.47 mmol) and HATU (42.4 mg, 0.11 mmol). The reaction solution was stirred for 30 min at 23 °C. The solvent was removed under reduced pressure and the crude product was purified by MPLC to yield the intermediate (40.6 mg, 0.07 mmol, 76%) as a red solid.

The intermediate (2.8 mg, 4.9 µmol) was dissolved in dry dichloromethane [DCM] (0.5 mL) and treated with TFA (0.1 mL). The reaction solution was stirred for 30 min at 23 °C. All volatiles were removed under reduced pressure and DCM (0.5 mL) was added. The solution was treated with DIPEA (8.5 µL, 0.05 mmol) and a solution of 2,5-dioxopyrrolidin-1-yl 1-(4-(6-methyl-1,2,4,5-tetrazin-3-yl)phenyl)-3-oxo-6,9,12,15,18-pentaoxa-2-azahenicosan-21-oate (3.0 mg, 4.9 µmol) in dry DCM (0.2 mL). The reaction solution was stirred for 1 h at 23 °C. The solvent was removed under reduced pressure and the crude product was purified by MPLC to yield the probe 2 (2.3 mg, 2.4 µmol, 49%) as a red solid.

### Examples of the invention:

### Example 1: Loading of azide functionalized CPG beads with CPT600

### Experimental procedure

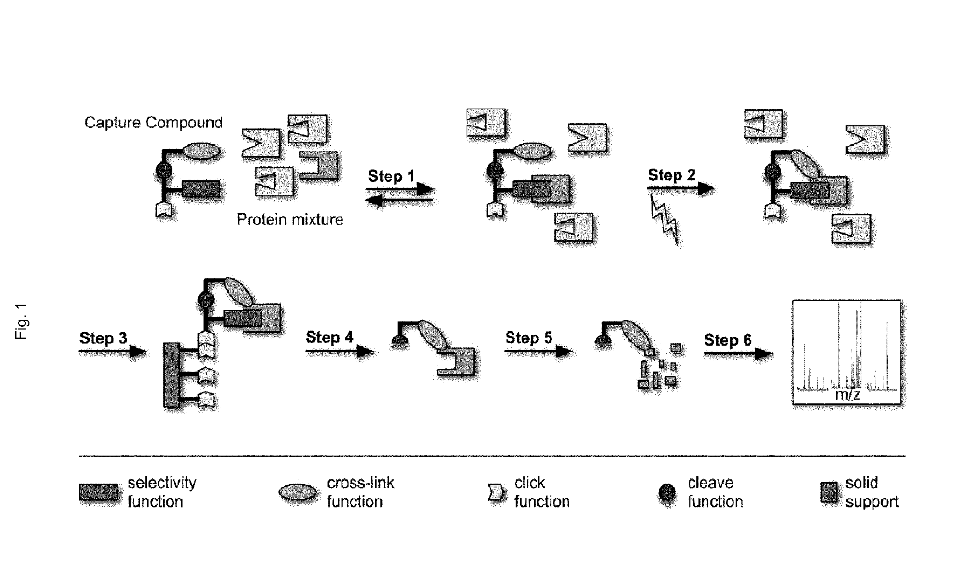

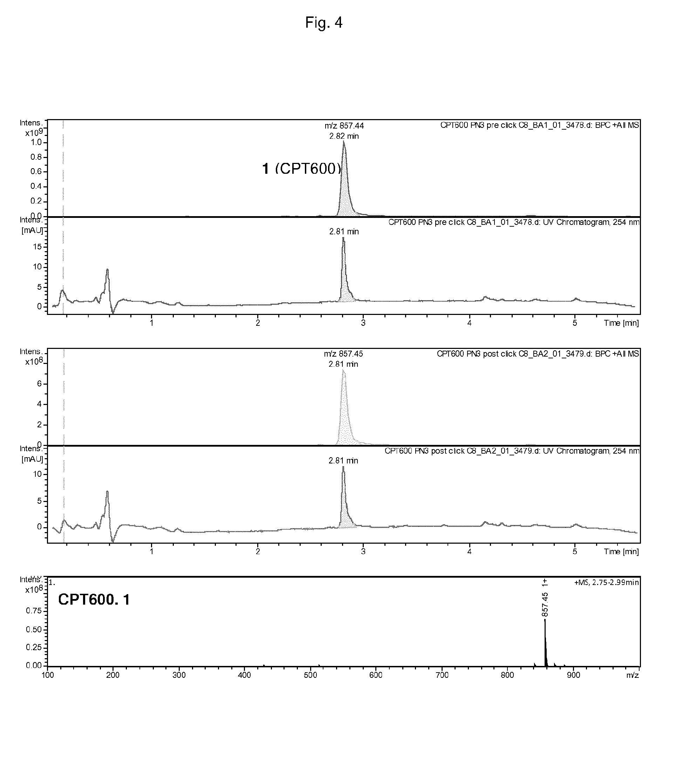

In a 2 mL Eppendorf reaction tube, 450 µL 10 g/l azide functionalized CPG beads (CPG-pN₃, Figure 1) in 20% EtOH were incubated with 275 µL 1.2 mM CPT600 (1, see structures below) in DMSO and 33 µL 5 mM CuSO₄ under rotation at r.t. (room temperature) for 25 min. After brief centrifugation (tabletop centrifuge), the supernatant was transferred into another 2 mL tube, 33 µL 50 mM sodium ascorbate was added, mixed and a sample (8.41 µl) was taken (pre-click, Figure 4). The remaining supernatant was mixed again with the CPG-pN3 and incubated under rotation at r.t for 1h. After brief centrifugation (tabletop centrifuge), a sample (8.41 µl) of the supernatant was taken (post-click, Figure 4) and the remaining supernatant was discarded. The CPG-pN3 were washed three times by re-suspending and centrifugating in 1.8 mL 20% EtOH, respectively and stored in 450 µL 20% EtOH at r.t. in the dark.

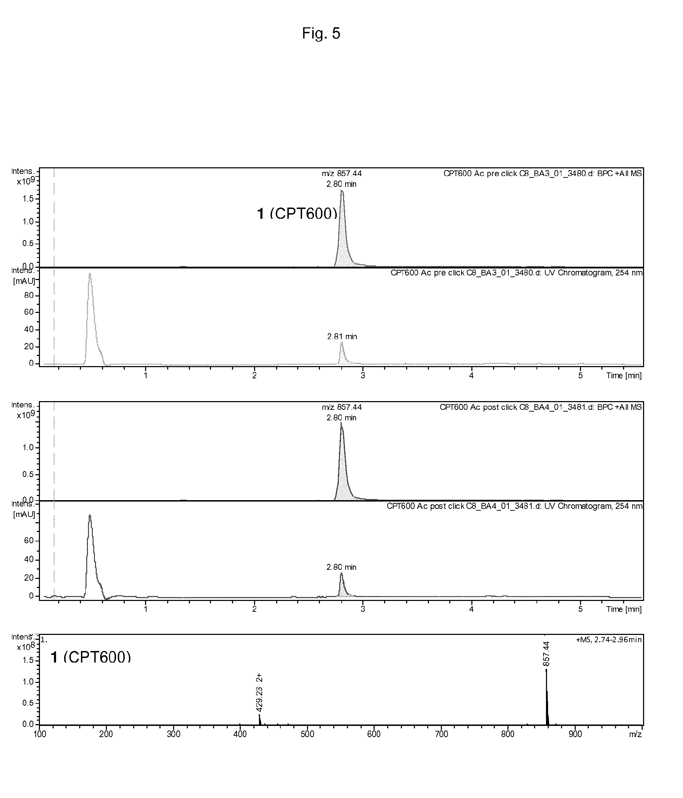

As a control, the same procedure was performed side-by-side using acetyl functionalized CPG beads (CPG-Ac) instead of CPG-pN3. Again, samples were drawn before (pre-click, Figure 5) and after (post-click, Figure 5) applying click conditions.

The four 8.41 µL samples were diluted with 330 µl 20% MeCN, respectively, and 10 µl, respectively, were separated via RP-HPLC on a YMC-Pack Octyl C8, 50x2.1 mm, 3 µm column and analyzed with UV and MS Instrumenmtation consisting of a Dionex Ultimate 3000 LC comprising a HPG-3200RS pump, WPS-3000TRS autosampler, TCC-3000RS column thermostat, and DAD-3000 UV detector coupled to a Bruker amaZon speed ion trap mass spectrometer governed by the Compass 1.5 software.

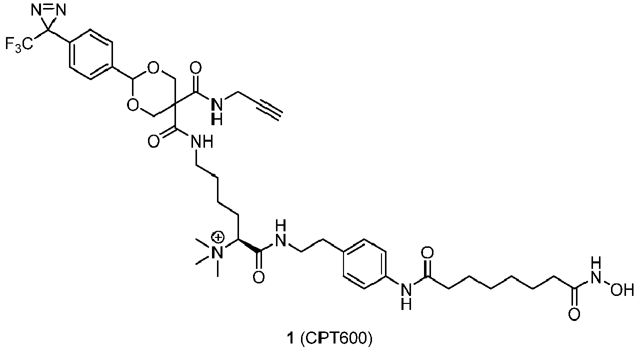

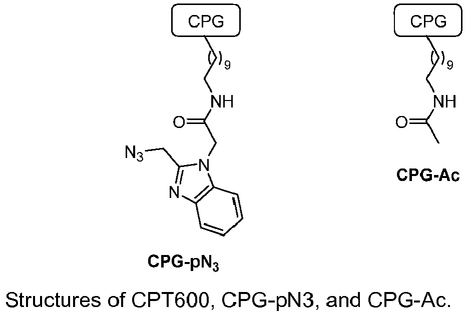

### Conclusion

Using CPG-pN₃, the amount of CPT600 decreases by about 10-15% (Figure 4, Table 1a), whereas using CPG-Ac, the amount of CPT600 is unaffected (Figure 5, Table 1b). Accordingly, about 33-50 nmol CPT600 have been loaded on 4.5 mg of CPG-pN3, resulting in about 7-11 nmol CPT600 / mg CPG-pN₃. The copper-catalyzed click reaction for functionalization of CPG-pN₃ is shown in Scheme 9.

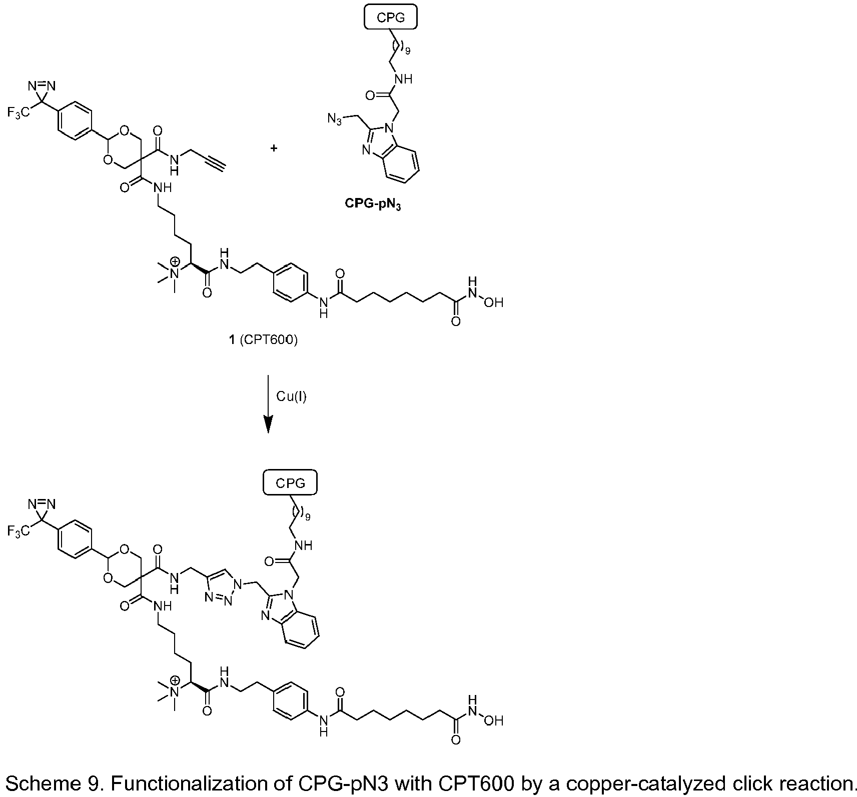

**Table 1a: Peak integration of Figure 4.**

| | **RT [min]** | **Chromatogram** | **Area** | **S/N** | **Max. m/z** |
|---|---|---|---|---|---|
| **pre** | 2.82 | BPC +All MS | 3728056320 | 968.3 | 857.44 |
| | 2.81 | UV Chromatogram, 254 nm | 38.895 | 6771.3 | |
| **post** | 2.81 | BPC +All MS | 3408637696 | 689.4 | 857.45 |
| | 2.81 | UV Chromatogram, 254 nm | 33.371 | 5673.5 | |
| % | | BPC +All MS | 91.43 | | |
| % | | UV Chromatogram, 254 nm | 85.8 | | |

**Table 1b. Peak integration of Figure 5.**

| | **RT [min]** | **Chromatogram** | **Area** | **S/N** | **Max. m/z** |
|---|---|---|---|---|---|
| **pre** | 2.8 | BPC +All MS | 6295347712 | 1372.5 | 857.44 |
| | 2.81 | UV Chromatogram, 254 nm | 70.292 | 12349.1 | |
| **post** | 2.8 | BPC +All MS | 6158362112 | 1370.1 | 857.44 |
| | 2.8 | UV Chromatogram, 254 nm | 73.283 | 12798 | |
| % | | BPC +All MS | 97.82 | | |
| % | | UV Chromatogram, 254 nm | 104.26 | | |

### Example 2: Cleavage of CPT600 after photo-reaction in solution

### Experimental procedure

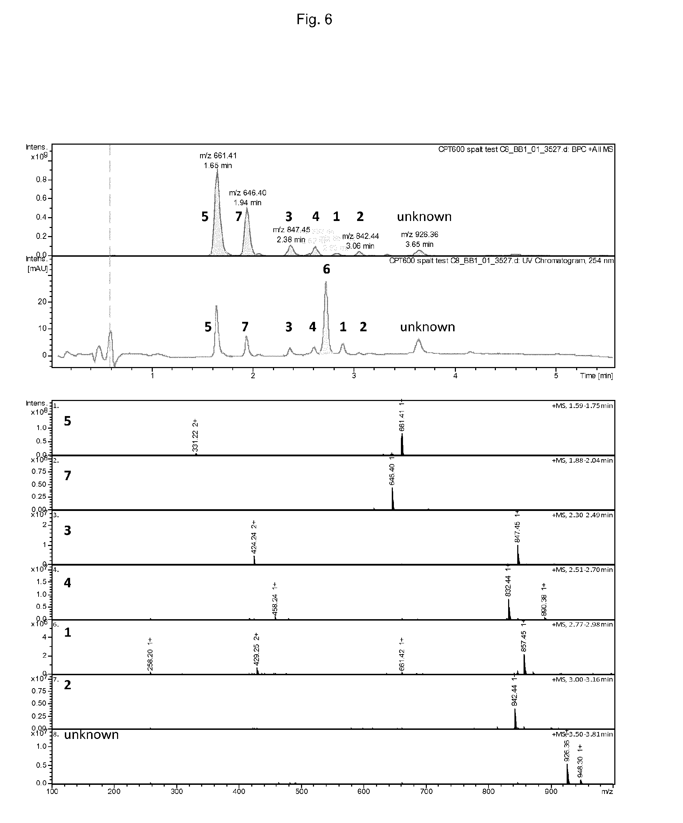

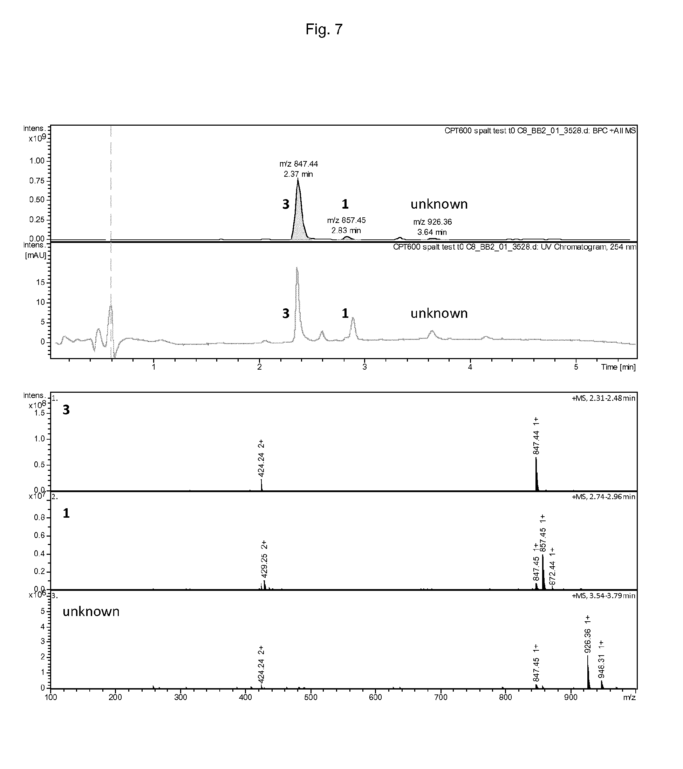

In a 100 µL PCR tube, 20 µL 73 µM CPT600 was irradiated at 4°C for 5 min in the caproBox (310 nm version, caprotec bioanalytics GmbH, Berlin, Germany). 1.7 µL 37% HCl was added, mixed and the solution was incubated on a thermoshaker at 37°C for 2 h. Then 100 µL Milli-Q water and 9 µL 2 N NaOH were added and mixed. The resulting solution as analyzed by RP-HPLC coupled to a UV detector and a mass spectrometer (Figure 6 and Table 2a) as described in example 1. As a control, the same sample but with only very brief incubation under acidic conditions (r.t. for about 10 s instead of 37°C for 2 h) was prepared (Figure 7 and Table 2b).

### Conclusion

UV irradiation of CPT600 (1) yields the reaction product of the resulting carbene (cleavage of N2) with water (3) (Figure 7, Table 2b, and Scheme 10). The reaction is almost complete, because only about 5% of the educt CPT600 (1) and more than 90% of the UV reaction product 3 is detected. About 3 % of an unknown impurity is detected.

Prolonged acidic treatment of CPT600 (1) after UV irradiation yields various products, all explainable by the hydrolysis of the acetal (5 and 7) and the hydroxamic acid (2, 4, and 7) (Figure 6, Table 2a, and Scheme 10). The main product is the fragment of 3 after acetal hydrolysis without hydroxamic acid hydrolysis (5). The associated fragment 6 was not detected in the MS chromatogram probably because of poor ionisability. In the UV chromatogram, a peak was detected that was later (example 4) characterized as fragment 6 by derivatization. Acetal hydrolysis is almost complete as estimated by the peak intensities of products after UV reaction with (5 and 7) and without (3 and 4) acetal hydrolysis. As observed for the short acid treatment control, a minor amount of the unknown impurity and the non-UV reacted CPT600 (1) is observed as well as its product after hydroxamic acid hydrolysis (2).

**Table 2a. MS-peak integration of Figure 6.**

| **Compound** | **RT [min]** | **Chromatogram** | **Area** | **Area Frac. %** | **S/N** | **Max. m/z** |
|---|---|---|---|---|---|---|
| **5** | 1.65 | BPC +All MS | 3429051648 | 51.68 | 421.6 | 661.41 |
| **7** | 1.94 | BPC +All MS | 1798498944 | 27.10 | 233.5 | 646.4 |
| **3** | 2.38 | BPC +All MS | 432395200 | 6.52 | 52.6 | 847.45 |
| **4** | 2.62 | BPC +All MS | 386267200 | 5.82 | 44.2 | 832.44 |
| **1** | 2.83 | BPC +All MS | 100943232 | 1.52 | 10.1 | 857.45 |
| **2** | 3.06 | BPC +All MS | 167125312 | 2.52 | 20.6 | 842.44 |
| **unknown** | 3.65 | BPC +All MS | 321067072 | 4.84 | 28.8 | 926.36 |

**Table 2b. MS-peak integration of Figure 7.**

| **Compound** | **RT [min]** | **Chromatogram** | **Area** | **Area Frac. %** | **S/N** | **Max. m/z** |
|---|---|---|---|---|---|---|
| **3** | 2.37 | BPC +All MS | 3091667200 | 91.56 | 917.9 | 847.44 |
| **1** | 2.83 | BPC +All MS | 171689952 | 5.08 | 48.9 | 857.45 |
| **unknown** | 3.64 | BPC +All MS | 113381904 | 3.36 | 26.4 | 926.36 |

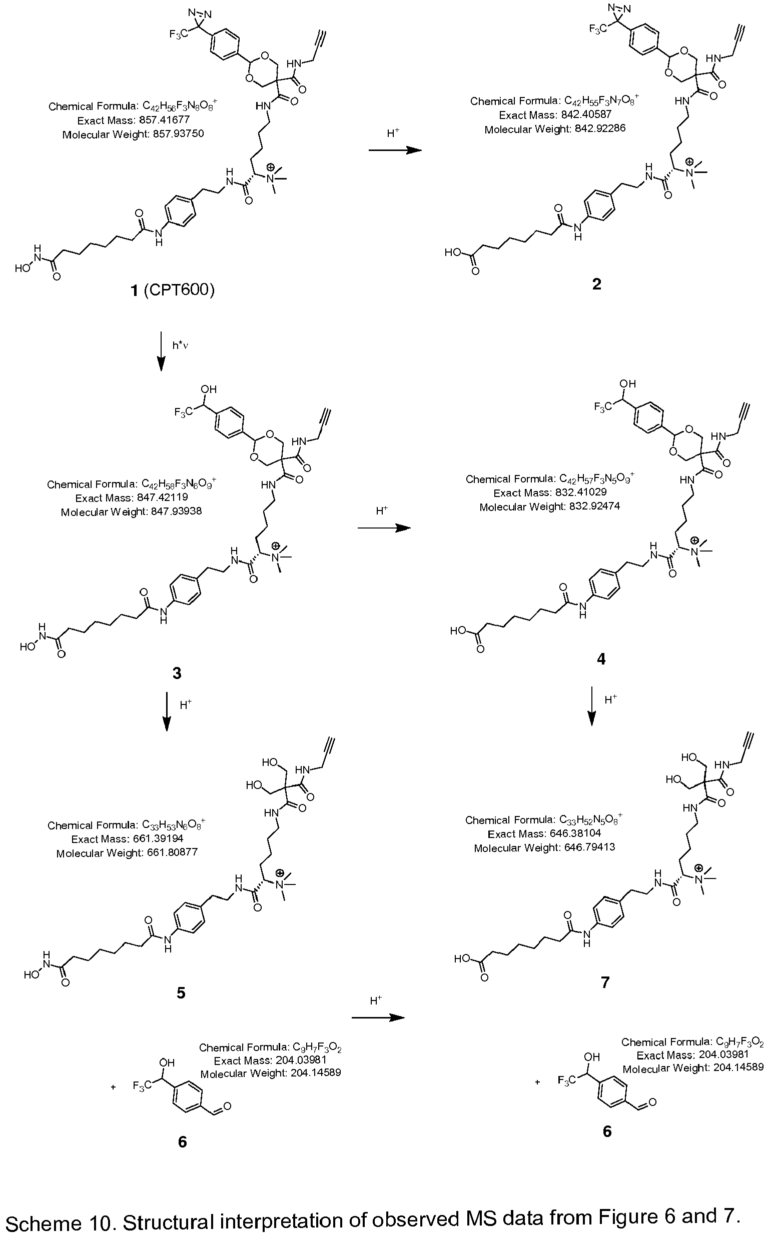

### Example 3: Cleavage of CPT600 after photo-reaction on CPG-pN₃

### Experimental procedure

20 µL 10 mg/mL CPT600-loaded CPG-pN3 from example 1 were transferred into a 100 µL PCR tube, centrifuged, the supernatant was discarded and 20 µL in water was added. The suspension was irradiated at 4°C for 5 min (with re-suspending every 30 s) in the caproBox (310 nm version, caprotec bioanalytics GmbH, Berlin, Germany). 1.7 µL 37% HCl was added, mixed and the solution was incubated on a thermoshaker at 37°C for 2 h. Then 100 µL Milli-Q water and 9 µL 2 N NaOH were added and mixed. After centrifugation, the resulting supernatant was analyzed by RP-HPLC coupled to a UV detector and a mass spectrometer as described in example 1. As a control, the same sample but with only very brief incubation under acidic conditions (r.t. for about 10 s instead of 37°C for 2 h) was prepared.

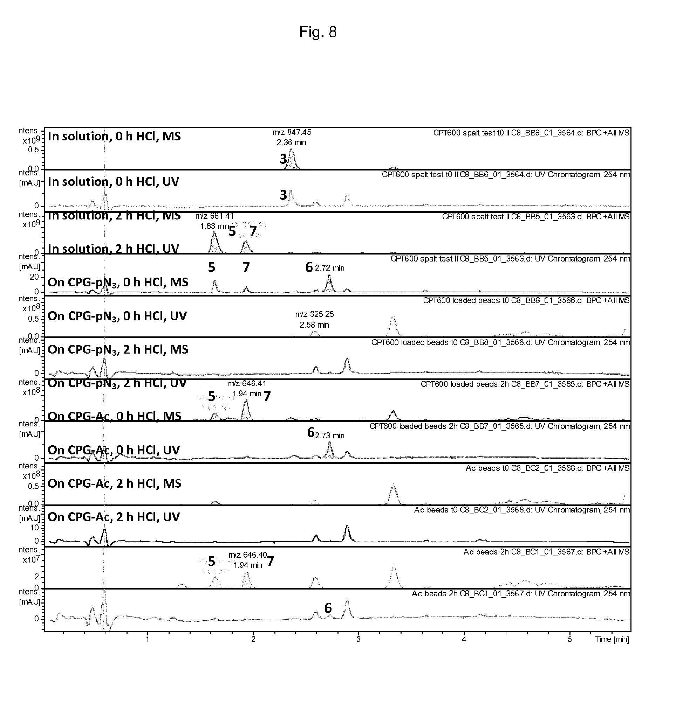

Further controls included using CPT600-treated CPG-Ac from example 1 (short and long acid treatment) instead of CPT600-loaded CPG-pN3 and CPT600 in solution (short and long acid treatment) as described in example 2. All six experiments were performed side-by-side. The results are summarized in Figure 8 and Table 3.

### Conclusion

The desired cleavage fragment 6 (see example 4 for verification) is clearly detected in the UV chromatogram of the UV-irradiated CPT600-loaded CPG-pN3 sample after 2 h acidic treatment (48.9% of the amount from the respective sample in solution as rated from the respective peak areas in the UV chromatograms). The respective sample employing CPG-Ac instead of CPG-pN3 only showed a minor amount of 6 (8.0% of the amount from the respective CPG-pN3 sample and 3.9% of the amount from the respective sample in solution as rated from the respective peak areas in the UV chromatograms).

Whereas after brief acidic incubation, no CPT600 derivative could be identified in the supernatant of the samples employing CPG beads, minor amounts of 5 and 7 (3.6% and 16.1%, respectively, compared to solution sample under the same conditions as rated from the respective peak areas in the base peak MS chromatograms) were identified after 2 h acidic treatment. Since 5 and 7 are present to about the same amount in the CPG-pN3 and CPG-Ac samples, these species probably arise from adsorption (non covalent binding) of a small proportion of 3 to the solid support (CPG surface).

**Table 3. MS-peak integration of Figure 8.**

| | **compound** | **RT [min]** | **Chromatogram** | **Area** | **Area Frac %** | **S/N** | **Max. m/z** |
|---|---|---|---|---|---|---|---|
| **In solution, 0 h HCl** | 3 | 2.36 | BPC +All MS | 211406284 8 | 100.00 | 892.1 | 847.45 |
| | 3 | 2.36 | UV 254 nm | 31.679 | 100.00 | 6147.1 | |
| **In solution, 2 h HCl** | 5 | 1.63 | BPC +All MS | 309716684 8 | 61.25 | 648.2 | 661.41 |
| | 7 | 1.94 | BPC +All MS | 179336780 8 | 35.46 | 381.1 | 646.4 |
| | 5 | 1.63 | UV 254 nm | 36.019 | 31.47 | 7939.5 | |
| | 7 | 1.93 | UV 254 nm | 15.084 | 13.18 | 3507.4 | |
| | 6 | 2.72 | UV 254 nm | 63.355 | 55.35 | 11932.7 | |
| **On CPG-pN3, 2 h HCl** | 5 | 1.64 | BPC +All MS | 111303352 | 25.23 | 28.8 | 661.42 |
| | 7 | 1.94 | BPC +All MS | 288188416 | 65.33 | 80.4 | 646.41 |
| | 6 | 2.73 | UV 254 nm | 30.9728 | 85.18 | 5810.6 | |
| **On CPG-Ac, 2 h HCl** | 5 | 1.65 | BPC +All MS | 86579064 | 31.13 | 26.8 | 661.42 |
| | 7 | 1.94 | BPC +All MS | 114680936 | 41.23 | 39.4 | 646.4 |
| | 6 | 2.73 | UV 254 nm | 2.4903 | 30.94 | 535 | |

### Example 4: Derivatization of the desired cleavage product aldehyde 6 to a hydrazone

### Experimental procedure

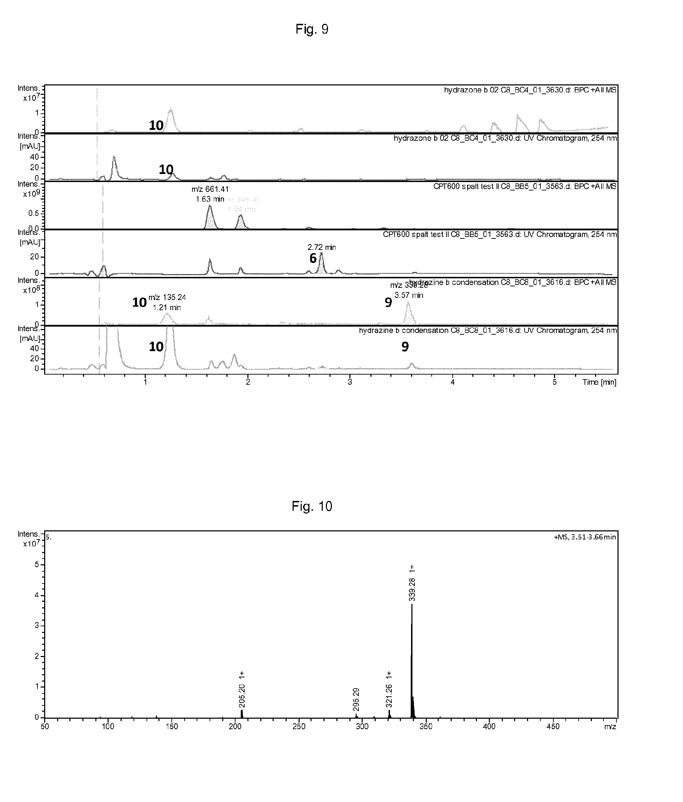

The final solution of example 2 (final 130 µL 11 µM CPT600 after photo-reaction with water, acid cleavage and quenching and dilution with NaOH) was analyzed by RP-HPLC as described in example 1 before and after addition of a 100-fold molar excess of hydrazine 8 (1.1 µL 125 mM 8 as its HCl salt in MeOH) (Figure 9 and Scheme 3).

### Conclusion

Photo-reacted and cleaved CPT600 shows an UV peak at retention time 2.72 min, which is largely diminished after addition of hydrazine 8 and a new signal is detected at retention time 3.57 min (Figure 5). The corresponding MS spectrum (Figure 10) suggests that aldehyde 6 has reacted with hydrazine 8 to hydrazone 9 (Scheme 11). When analyzing hydrazine 8 alone, the cyclic hydrazide 10 is detected as the major species in the MS chromatogram (Scheme 11).

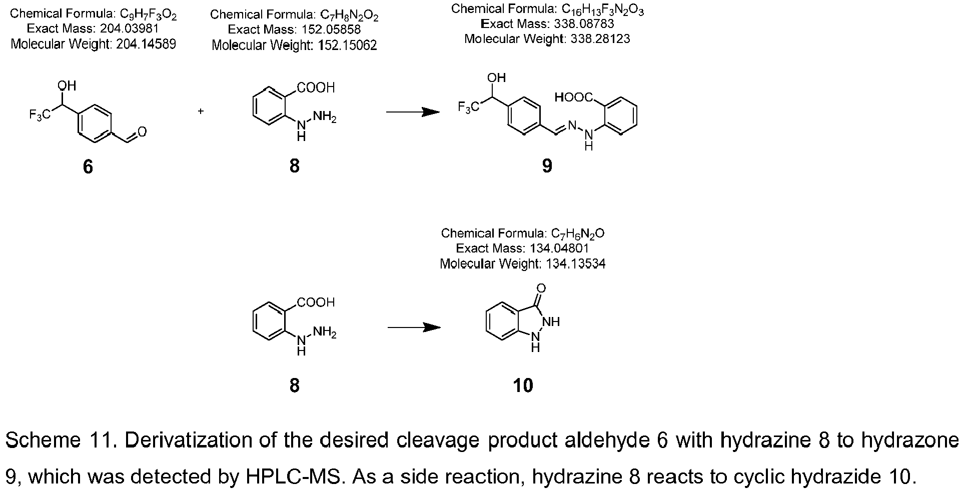

### Synthesis of capture compounds

### Synthesis of acetal building block 6 (Schema 12)

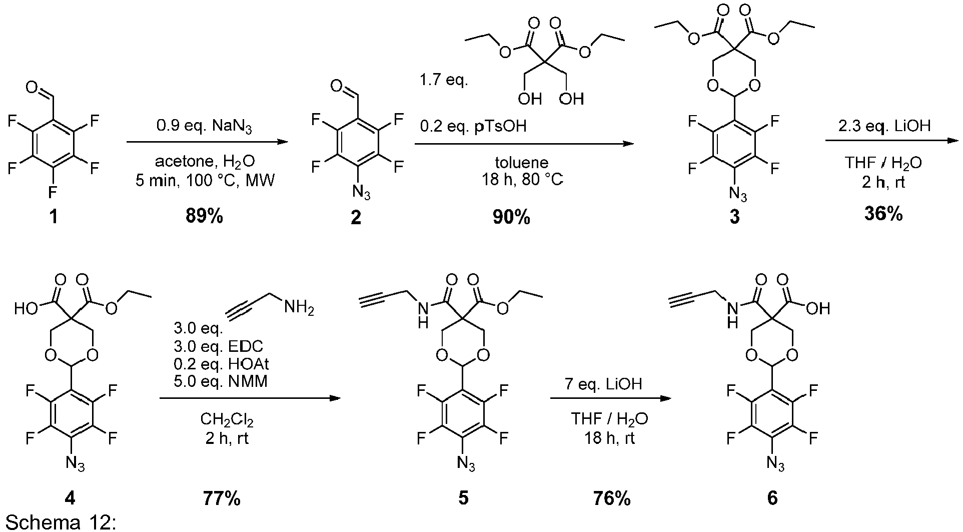

4-azido-2,3,5,6-tetrafluorobenzaldehyde (2): 545 mg of 2,3,4,5,6-pentafluorobenz-aldehyde (2) and 163 mg of NaN3 were given in one microwave flask (10-20 mL). 8 mL acetone and 2 mL water were added and reaction mixture was steered at room temperature until regents were dissolved. Flask was closed and reaction mixture was steered in microwave at 100 °C for 5 min.

30 mL water was added and reaktion mixture was extracted tree times with EtOAc (15 mL). The united organic extract was dried (MgSO4) and concentrated in vacuum to give 539 mg (89%) of the title compound as yellow solid.

diethyl 2-(4-azido-2,3,5,6-tetrafluorophenyl)-1,3-dioxane-5,5-dicarboxylate (3): 783 mg of pentafluorobenzaldehyde 2 and 1.34 g diethyl 2,2-bis(hydroxymethyl)malonate were dissolved in 40 mL Toluene. 123 mg of pTsOH was added and the reaction mixture was refluxed at 80 °C for 18 h. After this time 500 µL of Et3N were added and solvent was removed in vacuum. The residue was purified by column chromatography on silica gel (9 : 1 cyclohexane/EtOAc + 0.1 % Et3N) to afford 1.35 g (90%) of the title compound as colorless oil.

2-(4-azido-2,3,5,6-tetrafluorophenyl)-5-(ethoxycarbonyl)-1,3-dioxane-5-carboxylic acid (4): 1.35 g of acetal 3 were dissolved in 20 mL THF. 40 mL of water and 169 mg of LiOH were added. Reaction was steered at room temperature for 2.5 h. After the reaction was completed (TLC-test), 850 µL of 0.5 M HCl were added until the reaction mixture turned milky (pH = 3.0). Water phase was extracted with CH2Cl2 three times. The united organic extract was dried (MgSO4) and concentrated in vacuum to give 447 mg (36%) of the title compound as white solid.

ethyl 2-(4-azido-2,3,5,6-tetrafluorophenyl)-5-(prop-2-ynylcarbamoyl)-1,3-dioxane-5-carboxylate (5): To a solution of 153 mg of acetal 4 in 7 mL DCM, 75 µL of propargylamine were added. 224 mg EDC, 214 µL NMO and 10 mg HOAt were added successively. The reaction mixture was steered at room temperature for 1 h. After completion (LC-MS test) the reaction mixture was poured into the ice water and extracted twice with CH2Cl2. The united organic extract was dried (MgSO4) and concentrated in vacuum. The residue was purified by column chromatography on silica gel (3 : 2 cyclohexane/EtOAc) to afford 129 mg (77%) of the title compound as colorless oil.

2-(4-azido-2,3,5,6-tetrafluorophenyl)-5-(prop-2-ynylcarbamoyl)-1,3-dioxane-5-carboxylic acid (6): 129 mg of acetal 5 were dissolved in 1 mL THF. 2 mL of water and 50 mg of LiOH were added. Reaction mixture was steered at room temperature for 18 h. After the reaction was completed (TLC-test),0.7 mL of 0.5 M HCl was added to adduce pH between 3 and 4. Water phase was extracted with CH2Cl2 twice. The united organic extract was dried (MgSO4) and concentrated in vacuum to give 92 mg (76%) of the title compound as white solid.

### Synthesis of SAHA acetal capture compound 10 (Schema 13)

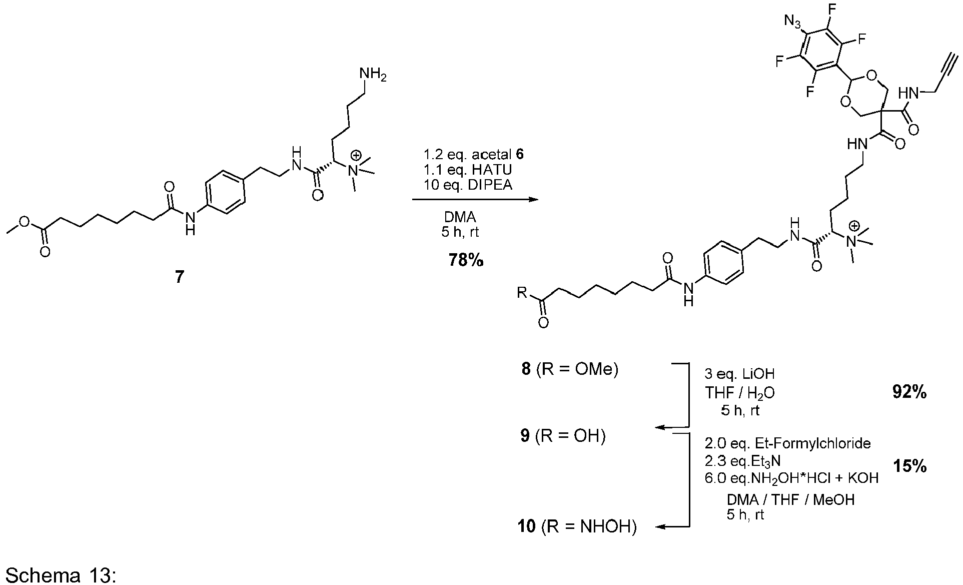

(S)-6-(2-(4-azido-2,3,5,6-tetrafluorophenyl)-5-(prop-2-ynylcarbamoyl)-1,3-dioxane-5-carboxamido)-1-(4-(8-methoxy-8-oxooctanamido)phenethylamino)-N,N,N-trimethyl-1-oxohexan-2-aminium (8): 37 mg (S)-6-amino-1-(4-(8-methoxy-8-oxooctanamido)phenethylamino)-N,N,N-trimethyl-1-oxohexan-2-aminium (7) was dissolved in 5 mL DMA and 37 mg of acetal 6 was added. After starting material was dissolved 33 mg HATU and 128 µL DIPEA were added to reaction mixture and steered 3 h at room temperature. After reaction was completed (LC-MS teat) DMA was removed in high vacuum and 1g of crude mixture was obtained. Crude product was purified by MPLC to give 52 mg (78%) of the title compound as lyophilized white solid.

(S)-6-(2-(4-azido-2,3,5,6-tetrafluorophenyl)-5-(prop-2-ynylcarbamoyl)-1,3-dioxane-5-carboxamido)-1-(4-(7-carboxyheptanamido)phenethylamino)-N,N,N-trimethyl-1-oxohexan-2-aminium(9): 52 mg of methyl ester 8 was dissolved in 2 mL THF and 2 mL water and 4.5 mg LiOH were added. Reaction mixture was steered for 2 h at room temperature. 2 M HCl was added to adduce the pH of the mixture between 6 and 7. THF was removed in vacuum and residue was lyophilized to give 47 mg (92%) of the title product as white solid.

(S)-6-(2-(4-azido-2,3,5,6-tetrafluorophenyl)-5-(prop-2-ynylcarbamoyl)-1,3-dioxane-5-carboxamido)-1-(4-(8-(hydroxyamino)-8-oxooctanamido)phenethylamino)-N,N,N-trimethyl-1-oxohexan-2-aminium(10): 52 mg of acid 9 was dissolved in 2 mL THF and 2 mL DMA in vacuum heated flask containing molecular sieves. This solution was cooled to 0° C and 11.2 µL ethyl formylchloride and 19 µL Et3N were added and the reaction mixture was steered for 30 min. In separate vacuum heated flask containing molecular sieves 25 mg hydroxylamine hydrochloride and 20 mg KOH were dissolved in 1.5 mL MeOH. After 15 min this mixture was filtrated using syringe filter and transferred into another vacuum heated flask containing molecular sieves. So generated NH2OH solution in MeOH was cooled down at 0 °C and the activated acid from the first flask was added to this solution slowly. Reaction mixture was steered 1 h at 0 °C and than 18 h at rt. 2 M HCl was added to adduce pH between 6 and 7, THF was removed in vacuum and residue was purified by MPLC to give 8 mg (15%) of the title compound as lyophilized white solid.

### Short description of the Figures:

- Fig.1: shows a standard work flow comprising the steps of: Step 1: interaction of the capture compound CC of the general formula 2 with proteins; Step 2: Photo-Cross-Link; Step 3: immobilizing and washing; Step 4: cleavage of the cleave function F; Step 5: (tryptic) digestion, Step 6: characterization (analytic);

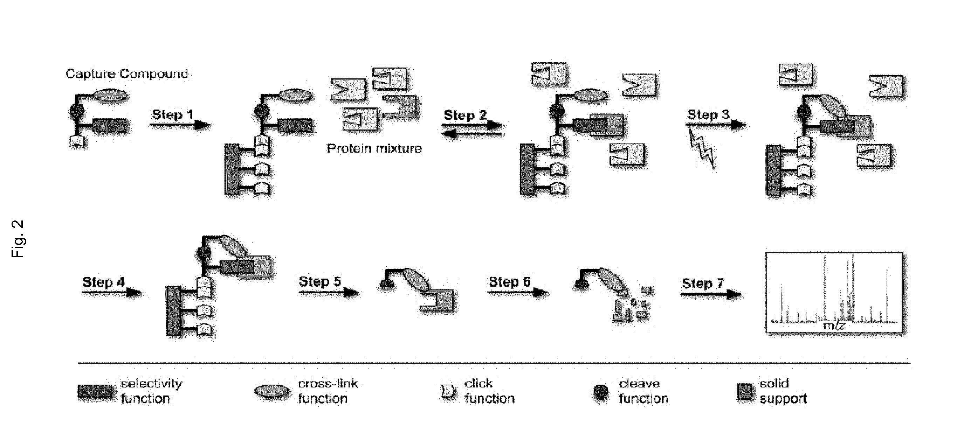

- Fig.2: shows a preclick work flow comprising the steps of: Step 1: immobilizing of the capture compound CC of the general formula 2; Step 2: interaction of the capture compound CC with proteins; Step 3: Photo-Cross-Link; Step 4: washing; Step 5: cleavage of the cleave function F; Step 6: (tryptic) digestion, Step 7: characterization (analytic);

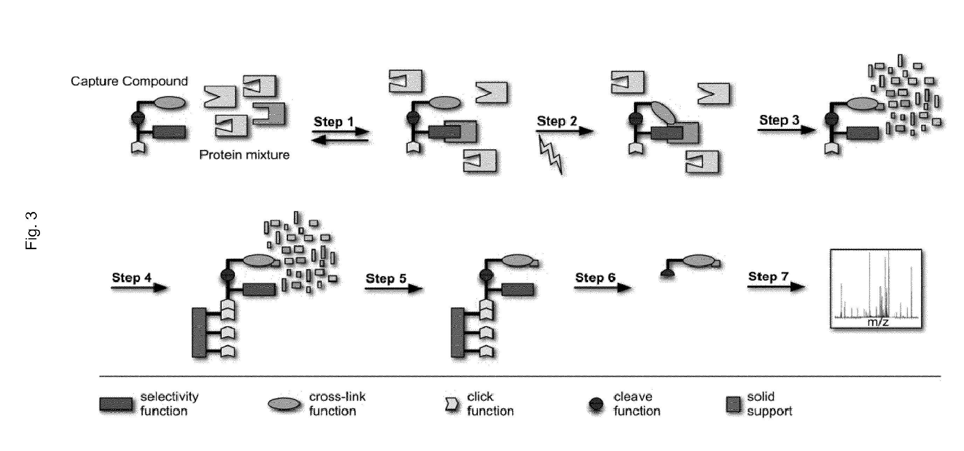

- Fig.3: shows a predigest work flow; comprising the steps of: Step 1: interaction of the capture compound CC of the general formula 2 with proteins; Step 2: Photo-Cross-Link; Step 3: (tryptic) digestion, Step 4: immobilizing of the capture compound CC; Step 5: washing; Step 6: cleavage of the cleave function F Step 7: characterization (analytic);
- Fig. 4: shows a base peak (MS) and UV (254 nm) chromatogram of a pre-click (top panels) and post-click (mid panels) example as well as MS spectrum (bottom panel) of CPT600 for loading CPG-pN₃;
- Fig. 5: shows a base peak (MS) and UV (254 nm) chromatogram of a pre-click (top panels) and a post-click (mid panels) example as well as MS spectrum (bottom panel) of CPT600 for the CPG-Ac control;
- Fig. 6: shows a base peak (MS) and UV (254 nm) chromatogram (top panels) and MS spectra (bottom panels) of the products resulting from CPT600 after UV irradiation and acid treatment; see Scheme 9 for structural interpretation;
- Fig. 7: shows a base peak (MS) and UV (254 nm) chromatogram (top panels) and MS spectra (bottom panels) of the products resulting from CPT600 after UV irradiation and very short acid treatment (control); see Scheme 9 for structural interpretation;
- Fig. 8: shows a base peak (MS) and UV (254 nm) chromatograms of the six samples;
- Fig. 9: shows a base peak (MS) and UV (254 nm) chromatograms of hydrazine 8 (upper two panels), photo-reacted and cleaved CPT600 (mid two panels), and photo-reacted and cleaved CPT600 after reaction with hydrazine 8 yielding hydrazone 9(lower two panels);
- Fig. 10: shows a MS spectrum of hydrazone 9.

## Claims

1. A process for isolating and/or characterising biomolecules and/or biomolecule fragments, in particular proteins and/or protein fragments, comprising the steps:
a. provision of an immobilized compound (IC) of the general formula (1) 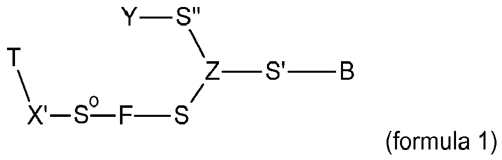 wherein
- T is a target compound comprising, in particular essentially consisting of, a biomolecule BM or a biomolecule fragment BMF, more particularly a protein P or a protein fragment PF,
- X' is the chemical structure resulting from activation of the reactivity function X by irradiation and the subsequent forming of a covalent bond to the target compound T,
- F is a cleavable function,
- Y is a selectivity function selected from the group comprising a bioactive small molecule,
- Z is carbon, nitrogen, phosphorus, silicon, sulfonium, cycloalkyl, unsaturated cycloalkyl, unsaturated heterocycloalkyl or heteroaryl,
- B is a macroscopic carrier, which is capable of being separated from a medium by means of his physical properties,
- S°, S, S' and S" are spacer moieties, each of which represents, independently of the others, a bridge between X' and F, Z and F, Z and B or Z and Y, facilitated by covalent bonds of carbon, nitrogen, oxygen, sulphur, silicon and/or phosphorous atoms, wherein S^{o}, S and S" are optional, and wherein S' comprises the spacer moieties S'" and S^{iv} of formula 2 and 3 and additionally the structural element derived from the reaction of the linking functions D and E;
through the interaction of
i. a capture compound (CC) of the general formula 2 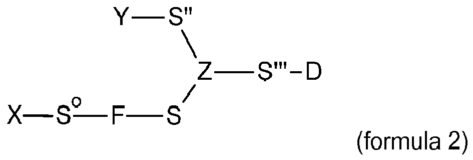 wherein
- X is a reactivity function which can be activated by irradiation and can form after said activation a covalent bond to the target compound T, and
- F, Y, Z, S, S° and S" each have the meanings defined previously, and
- S'" is a spacer moiety representing a bridge between Z and D, facilitated by covalent bonds of carbon, nitrogen, oxygen, sulphur, silicon and/or phosphorous atoms, wherein S'" is optional, and
- D is a first linking function capable of forming a covalent bond selectively with a second linking function E under reaction conditions not leading to a covalent reaction of D or E with natural occurring polypeptides, in particular with proteins;
ii. the target compound T; and
iii. a carrier compound CCB of the general formula 3 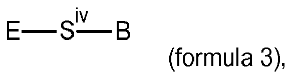 wherein
- S^{iv} is a spacer moiety representing a bridge between E and B facilitated by covalent bonds of carbon, nitrogen, oxygen, sulphur, silicon and/or phosphorous atoms, wherein S^{iv} is optional,
- B has the meaning defined previously, and
- E is said second linking function; and
b. carrying out a division step, in which the cleavable function F is cleaved, and a division compound DC of the general formula 4 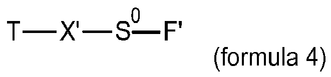 is produced, wherein
- T, S⁰ and X' have the meanings defined previously, and
- F' is the chemical structure resulting from a cleavage reaction of the cleavable function F; and
c. isolation and/or characterisation of said division compound (DC).

2. The process according to claim 1, wherein the provision of an immobilized compound (ICa), wherein T is the at least one biomolecule BM, in particular the at least one protein P, is achieved by means of the following steps:
a. carrying out a bonding step, wherein a biological sample comprising one or more proteins is brought into contact with the capture compound (CC) of formula (2) under conditions leading to a selective interaction of said selectivity function Y with at least one biomolecule BM or at least one protein P of the biological sample, and subsequently
b. an activation of the reactivity function X by the effect of irradiation is carried out, wherein subsequently said reactivity function X forms a covalent bond with BM or P yielding the function X', producing a protein capture compound (PCC) of the general formula (5) or (5'), in particular 5', 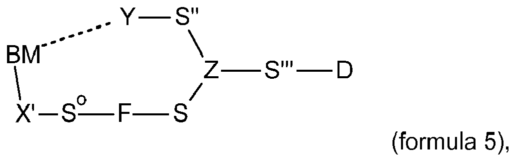 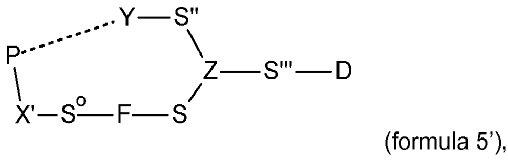 and subsequently
c. carrying out a further bonding step, wherein the protein capture compound (PCC) of formula (5) or (5') is brought into contact with the carrier compound (CCB) of formula (3) under conditions resulting in a covalent bond between D and E, producing the immobilized compound (ICa) of the general formula (1a) or (1a'), in particular 1a', 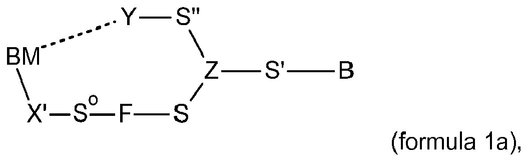 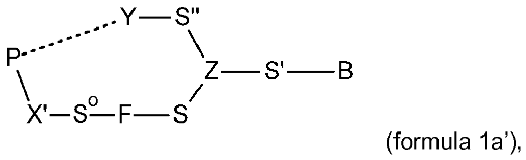 and subsequently
d. isolation of the immobilized compound (ICa) of formula (1 a) or (1a'),
wherein X', Z, P, BM, S^{o}, S, S",S"', S^{iv}, Y, B, D, E and F have the meanings given previously, and wherein S' comprises the spacer moieties S'" and S^{iv} of formula (3) and (5) or (5') and additionally the structural element derived from the reaction of the linking functions D and E.

3. The process according to claim 1, wherein the provision of an immobilized compound (ICa), wherein T is the at least one biomolecule BM, in particular the at least one protein P, is achieved by means of the following steps:
a. carrying out a bonding step, wherein the carrier compound (CCB) of formula (3) is brought into contact with the capture compound (CC) of formula (2) under conditions resulting in a covalent bond between D and E, producing a precursor compound (PC) of the general formula (6) 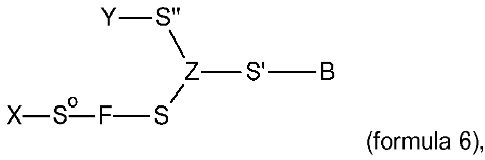 and subsequently
b. carrying out a further bonding step, wherein a biological sample comprising one or more proteins is brought into contact with the precursor compound (PC) of formula (6) under conditions leading to a selective interaction of said selectivity function Y with at least one biomolecule BM or the at least one protein P from the biological sample; and subsequently
c. an activation of the reactivity function X by the effect of irradiation is carried out, wherein subsequently said reactivity function X forms a covalent bond with BM or P yielding the function X', producing the immobilized compound (ICa) of formula (1 a) or (1 a'), and subsequently
d. isolation of the immobilized compound (ICa) of formula (1 a) or (1a'),
wherein X, X', Z, P, S°, S, S', S",S"', S^{iv}, Y, B, D, E and F have the meanings given previously.

4. The process according to any one of the claims 2 or 3, wherein
a. before the division step b of claim 1, the at least one biomolecule BM, in particular the at least one protein P, of the compound of formula (1 a) or (1 a') is broken down into individual fragment parts in a digestive step, producing an immobilized compound (ICb) of the general formula (1 b) or (1 b'), in particular(1b') 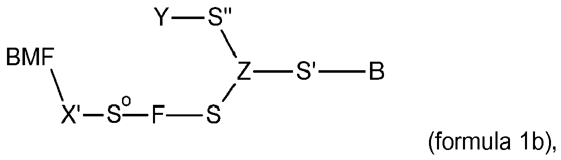 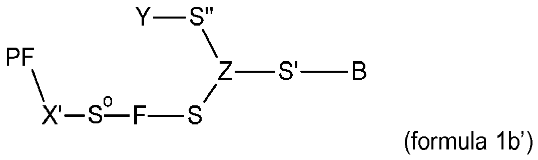 wherein
- X', Z, P, BMF, S°, S, S', S", Y, B and F have the meanings defined previously, and
- BMF or PF are the fragment parts of the at least one digested biomolecule BM or the at least one digested protein P which are bound to the immobilising compound (ICb) of formula (1 b) or (1 b') by the function X'; and subsequently
b. a division step is carried out, in which the cleavable function F of the compound of formula (1 b) or (1 b') is cleaved, producing a division compound (DCb) of the general formula (4b) or (4b'), in particular (4b') 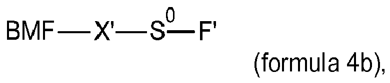 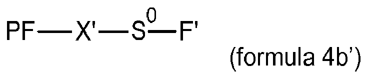 wherein
- BMF, PF, S⁰ and X' have the meanings defined previously, and
- F' is the chemical structure resulting from a cleavage reaction of the cleavable function F; and subsequently
c. an isolation and/or a characterisation step of said division compound (DCb) of formula (4b) or (4b') is carried out or a characterisation step of said division compound DCb of formula 4b or 4b' and the further fragment parts of the at least one digested biomolecule BM or the at least one digested protein P, which are not bound to the immobilising compound ICb of formula 1 b or 1 b', is carried out.

5. The process according to any one of the claims 2 or 3, wherein
a. a compound of the formula (1 a) or (1 a') is provided and a division step is carried out, in which the cleavable function F is cleaved, producing a division compound DCa of the general formula (4a) or (4a'), in particular (4a'), 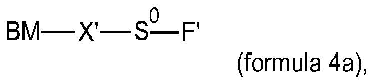 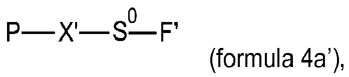 and subsequently an isolation and/or a characterisation step of said division compound (DCa) of formula (4a) or (4a') is carried out ,
or
b. before the characterisation of step a, the at least one biomolecule BM or the at least one protein P of the compound of the formula (4a) or (4a') is broken down into individual fragment parts BMF or PF in a digestive step, producing a division compound (DCb) of the general formula (4b) or (4b'), in particular (4b'), 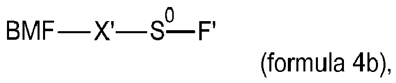 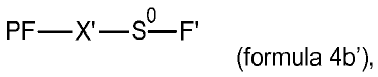 and subsequently an isolation and/or a characterisation step of said division compound (DCb) of formula (4b) or (4b') is carried out or a characterisation step of said division compound DCb of formula 4b or 4b' and the further fragment parts of the at least one digested biomolecule BM or the at least one digested protein P, which are not bound to the immobilising compound ICb of formula 1 b or 1 b', is carried out,
wherein
- F', S⁰ and X' have the meanings defined previously, and
- BMF or PF are the fragment part of the at least one digested biomolecule BM or the at least one digested protein P which are bound to the division compound (DCa) or (DCb) of formula (4a), (4a'), (4b) or (4b') by the function X'.

6. The process according to claim 1, wherein an immobilized compound (IC) is provided by means of the following steps:
a. carrying out a bonding step, wherein a biological sample comprising one or more proteins is brought into contact with the capture compound (CC) of formula (2) under conditions leading to the selective interaction of said selectivity function Y with the at least one biomolecule BM or the at least one protein P of the biological sample; and subsequently
b. an activation of said reactivity function X by the effect of irradiation is carried out, wherein subsequently said reactivity function X forms a covalent bond with BM or P producing a protein capture compound (PCC) of the general formula (5) or (5'), in particular (5'), 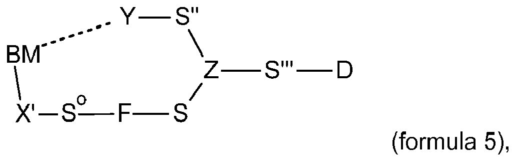 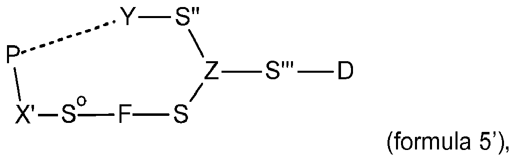 and subsequently
c. a digestive step is carried out, wherein said at least one biomolecule BM or at least one protein P of the compound of formula (5) or (5') is broken down into individual fragment parts producing a protein fragment capture compound (PFCC) of the general formula (5a) or (5a'), in particular (5a'), 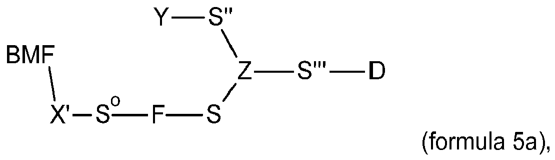 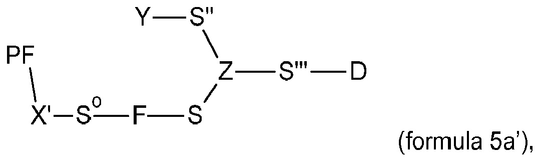 and subsequently
d. carrying out of a further bonding step, wherein the protein fragment capture compound (PFCC) of formula (5a) or (5a') is brought into contact with the carrier compound (CCB) of formula (3) under conditions resulting in a covalent bond between D and E producing the immobilising compound (ICb) of formula (1 b) or (1 b'), and subsequently
e. isolation of the immobilising compound (ICb) of the general formula (1b) or (1b'),
wherein X, X', Z, P,BM, BMF, PF, S^{o}, S, S', S", S"', S^{iv}, Y, B, D, E, F' and F have the meanings defined previously.

7. The process according to claim 6, wherein
a. a division step is carried out on the immobilising compound (ICb) of the general formula (1 b) or (1 b'), in which the cleavable function F is cleaved, producing a division compound (DCb) of the general formula (4b) or (4b'), in particular (4b') 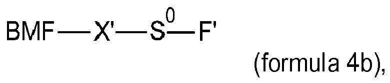 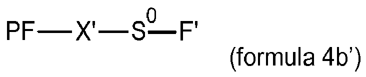 wherein
BMF, PF, S⁰ and X' have the meanings defined previously, and
- F' is the chemical structure resulting from a cleavage reaction of the cleavable function F; and subsequently
b. an isolation and/or a characterisation step of said division compound (DCb) of formula (4b) or (4b') is carried out.

8. The process according to at least one of the preceding claims, wherein
- Y is a bioactive small molecule selected from the group comprising a pharmaceutical drug, a drug development candidate, a drug fragment, a drug metabolite, a prodrug, a herbicide, a fungicide, an insecticide or a natural product, wherein in particular all the before mentioned selectivity functions Y are small molecular compounds selected from the group of a pharmaceutical drug, a drug fragment, a drug metabolite, a prodrug, a herbicide or a natural product with less than 1500 u, in particular less than 1000 u, more particularly less than 500 u, in molecular mass, and/or
- F is a cleavable function, which is cleavable by H⁺, OH⁻, thiols, salts from acids, fluorides, hydrazides, nucleophiles, electrophiles, radicals, alkylating agents, oxidation, reduction, enzymes or irradiation or a combination of the before mentioned, in particular F is selected from the group comprising an azo-, an acyl-, a disulfide-, a silyoxy-, a carbamate- , an acetal-, a ketal, an arylether, a hydrazone-, an arylketone- or a o-nitrobenzyl group; and/or
- X is selected from a diazirine-, an arylazide-, a di- or tetrahalogenarylazide or an arylketone group, wherein X is selected particularly from the group comprising aryltrifluoromethyldiazirine, tetrahalogenarylazide; and/or
- S^{o}, S, S', S", S'" and/or S^{iv} comprise an aliphatic chain or a heteroalkylchain, which comprises carbon and nitrogen, oxygen, sulphur, silicon and/or phosphorous atoms, wherein in particular said heteroalkyl chain comprises at least one oxygen atom, optionally the aliphatic chain or the heteroalkylchain comprise one or more carbon double or triple bonds.

9. The process according to at last one of the preceding claims, wherein D and E are each selected together from the group of reaction partners, which are capable of performing a reaction according to the click chemistry, wherein in particular
c. the reaction partners comprise
- an azide for one reaction partner and
- a member of the group comprising alkyne, aryl carboxylic acid methylester- ortho-phosphine, phosphite ester or a chelatising ligand for the other reaction partner or
d. the reaction partners comprise
- a triple bond or a double bond, particularly a cyclic double bond, such as for example norbornene or cyclooctene or a derivative thereof for one reaction partner, and
- a tetrazine or tetrazine derivative for the other reaction partner.

10. The process according to at least one of the preceding claims, wherein
- B comprises
- a polymer material or monolithic polymer material;
- monolithic silica or polymer silica material;
- CPG; and/or
- B comprises magnetic properties, in particular a magnetic particle.

11. The process according to at least one of the preceding claims, wherein X, X', F, F' and/or S° comprise a marker, in particular an isotopic marker, a mass tag, fluorescent tag and/or a multiplex marker.

12. The process according to at least one of the preceding claims, wherein the characterization step is achieved by applying mass spectrometry concept, in particular matrix assisted laser desorption ionization (MALDI), continuous or pulsed electrospray ionization (ESI), ionspray, thermospray, or massive cluster impact mass spectrometry for ionization, and linear time-of-flight (TOF), reflectron time-of-flight, single quadrupole, multiple quadrupole, single magnetic sector, multiple magnetic sector, Fourier transform ion cyclotron resonance (ICR), orbitrap or ion trap for mass analysis.

13. A compound, particularly a compound for carrying out a process according to at least one of the claims 1 to 12, of the general formula 3, 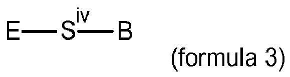 wherein
- S^{iv} comprises a polyethylene glycol group,
- B is a macroscopic carrier, in particular B comprises CPG, monolithic silica, polymer silica or a polymer material, a monolithic polymer material, wherein in particular B comprises additionally magnetic properties, and
- E comprises a carbon-carbon double bond or a carbon-carbon triple bond, in particular a cyclic double bond, such as for example norbornene or cyclooctene, tetrazine or a derivative of the aforementioned compounds.

14. A compound, particularly a compound for carrying out a process according to at least one of the claims 1 to 12, of the general formula 2, 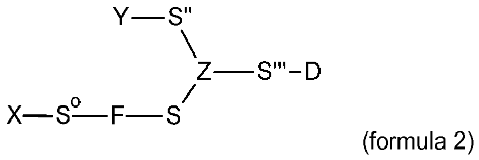 wherein
- X is a reactivity function which can be activated by irradiation and can form after said activation a covalent bond to a target compound T,
- F is a cleavable function,
- Y is a selectivity function selected from the group comprising a bioactive small molecule,
- Z is carbon, nitrogen, phosphorus, silicon, sulfonium, cycloalkyl, unsaturated cycloalkyl, unsaturated heterocycloalkyl or heteroaryl,
- D is a first linking function capable of forming a covalent bond selectively with a second linking function E under reaction conditions not leading to a covalent reaction of D or E with natural occurring polypeptides, in particular with proteins,
- S°, S, S" and S'" are spacer moieties, each of which represents, independently of the others, a bridge between X and F, Z and F, Z and Y or Z and D, facilitated by covalent bonds of carbon, nitrogen, oxygen, sulphur, silicon and/or phosphorous atoms, wherein S^{o}, S, S" and S'" are optional.

15. A compound, particularly a compound for carrying out a process according to at least one of the claims 1 to 12, of the general formula 6, 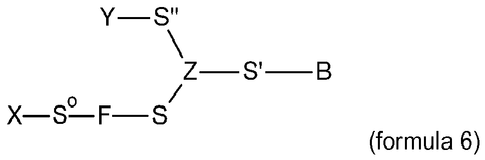 wherein
- X is a reactivity function which can be activated by irradiation and can form after said activation a covalent bond to a target compound T,
- F is a cleavable function,
- Y is a selectivity function selected from the group comprising a bioactive small molecule, in particular
- Y is bioactive small molecule selected from the group comprising a pharmaceutical drug, a drug development candidate, a drug fragment, a drug metabolite, a prodrug, a herbicide, a fungicide, an insecticide or a natural product, wherein in particular all the before mentioned selectivity functions Y are low-molecular weight compounds, more particularly a pharmaceutical drug, a drug fragment, a drug metabolite, a prodrug, a herbicide or a natural product with less than 1500 u, in particular less than 1000 u, more particularly less than 500 u, in molecular mass,
- Z is carbon, nitrogen, phosphorus, silicon, sulfonium, cycloalkyl, unsaturated cycloalkyl, unsaturated heterocycloalkyl or heteroaryl,
- B is a macroscopic carrier, which is capable of being separated from a medium by means of his physical properties,
- S°, S, S' and S" are spacer moieties, each of which represents, independently of the others, a bridge between X and F, Z and F, Z and B or Z and Y, facilitated by covalent bonds of carbon, nitrogen, oxygen, sulphur, silicon and/or phosphorous atoms, wherein S°, S and S" are optional and wherein S' comprises the spacer moieties S'" and S^{iv} of formula 2 and 3 and additionally the structural element derived from the reaction of the linking functions D and E.

16. A compound, particularly a compound obtainable by a method with the features of at least one of the claims 1 to 12, of the general formula 4, 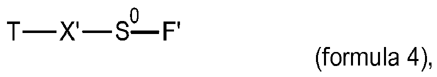 in particular a compound of the general formula (4b) or (4b'), more particularly (4b'), 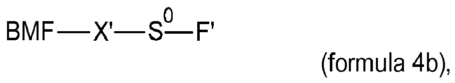 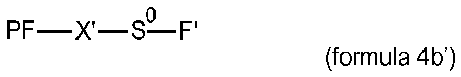 wherein
- X' is the chemical structure resulting from activation of the reactivity function X by irradiation and the subsequent forming of a covalent bond to the target compound T,
- T is a target compound comprising, in particular essentially consisting of, a biomolecule BM or a biomolecule fragment BMF, more particularly of a protein P or a protein fragment PF,
- S° is a spacer moiety, which represents a bridge between X' and F', facilitated by covalent bonds of carbon, nitrogen, oxygen, sulphur, silicon and/or phosphorous atoms, wherein S° is optional,
- F' is the chemical structure resulting from a cleavage reaction of a cleavable function F, wherein F is a cleavable function, which is cleavable by H⁺, OH⁻, thiols, salts from acids, fluorides, hydrazides, nucleophiles, electrophiles, radicals alkylating agents, oxidation, reduction, enzymes or irradiation or a combination of the before mentioned, in particular F is selected from the group comprising an azo-, an acyl-, a disulfide-, a silyoxy-, a carbamate- , an acetal-, a ketal, an arylether, a hydrazone-, an arylketone or a o-nitrobenzyl group.
